# EUROPEAN PATENT SPECIFICATION

(11) **EP 1 190 248 B1**
(45) Date of publication and mention of the grant of the patent: **24.08.2005**
(21) Application number: 00937135.2
(22) Date of filing: 27.06.2000
(51) Int. Cl.: G01N 33/50, G01N 33/68, C12N 9/02, C12Q 1/26, A61K 7/48

(54) **SCREENING METHODS FOR COMPOUNDS WHICH AFFECT MELANOGENESIS**
SCREENINGVERFAHREN FÜR VERBINDUNGEN WELCHE DIE MELANOGENESE BEEINFLUSSEN
PROCEDES DE CRIBLAGE DE COMPOSES AYANT UNE INCIDENCE SUR LA MELANOGENESE

(30) Priority: 29.06.1999 US 141563 P
(43) Date of publication of application: 27.03.2002
(73) Proprietor: NEW YORK UNIVERSITY, New York, NY 10012 (US)
(72) Inventor: ORLOW, Seth, J., New York, NY 10016 (US); MANGA, Prashiela, New York, NY 10016 (US)
(74) Representative: Grund, Martin
(86) International application number: PCT/IB2000/000861
(87) International publication number: WO 2001/001131

(56) References cited:
- US-A- 5 628 987
- PATENT ABSTRACTS OF JAPAN vol. 1996, no. 04, 30 April 1996 (1996-04-30) & JP 07 324095 A (HIDEJI ITOKAWA ET AL.), 12 December 1995 (1995-12-12)
- MEDLINE Washington DC USA; abstract no. 20133868, abstract XP002151889 & W.S. OETTING ET AL.: "Mutations of the human P gene associated with Type II oculocutaneous albinism (OCA2)" HUMAN MUTATION, vol. 12, no. 6, 1998, page 434 Minneapolis MN USA
- CHEMICAL ABSTRACTS, vol. 126, no. 25, 23 June 1997 (1997-06-23) Columbus, Ohio, US; abstract no. 328544, XP002151890 & D.N. HU ET AL.: "Regulation of melanogenesis by human uveal melanocytes in vitro" EXPERIMENTAL EYE RESEACH, vol. 64, no. 3, 1997, pages 397-404, New York NY USA

## Description

### 1. Field of the Invention

The invention is in the fields of cell biology, drug discovery, and cosmetics. Methods of screening compounds that can affect P protein function are provided. The invention further relates to methods of using such compounds for the cosmetic and therapeutic reduction or increase of melanin content in human and animal cells.

### 2. Background of the Invention

Melanin is a dark pigment found in plants and animals that protects against ultraviolet radiation and provides decoration in the skin, eyes, hair, and fur of animals (reviewed in Riley, P.A., 1997, Int. J. Biochem. Cell Biol. 11:1235-39). There are two different types of melanin: brown/black eumelanin and yellow/red pheomelanin. Melanocytes are cells of the epidermis specialized to produce melanin. A sophisticated intercellular signaling system determines whether an individual melanocyte will produce eumelanin or pheomelanin (reviewed in Brilliant, M.H. and Barsh, G.S., 1998, in *The Pigmentary System: Physiology and Pathophysiology*, 217-29, Oxford University, New York (Nordlund, J.J. *et al.,* eds)).

Melanocytes synthesize melanin inside of specialized organelles called melanosomes (reviewed in Orlow, S.J., 1998, in *The Pigmentary System: Physiology and Pathophysiology,* 97-106, Oxford University, New York (Nordlund, J.J. *et al*., eds)). Melanosomes are formed by the fusion of two types of vesicles. One type of vesicle, called a premelanosome, apparently derives directly from either the smooth endoplasmic reticulum or the *trans*-Golgi network. The other type of vesicle derives from the *trans*-Golgi network. Each of these types of vesicles contributes proteins to the melanosome necessary for its function.

Defects in the production of melanin result in pigmentation deficiencies such as albinism. Genetic analysis of abnormally pigmented strains of mice has identified more than 60 genes necessary for the normal production of melanin (reviewed in Silvers, W.K., 1979, *The Coat Colors of Mice: A Model for Mammalian Gene Action and Interaction*, Springer-Verlag, Basel). One of these genes encodes the enzyme tyrosinase. Tyrosinase protein is a multi-functional enzyme that catalyzes several steps in the production of melanin; tyrosinase activities include the rate-limiting steps of converting tyrosine to dihydroxyphenylalanine (DOPA), and DOPA to dopaquinone (reviewed in Lerner, A.B., and Fitzpatrick, T.B., 1950, Physiol. Rev. 30:91-126), as well as the oxidation of 5,6-dihydroxyindole to 5,6-indolequinone (Korner and Pawelek, 1982, Science 217:1163-1165). Both humans and mice lacking tyrosinase activity suffer a severe form of albinism.

Two tyrosinase-related proteins (TRP-1, encoded by the mouse *brown* gene, and TRP-2, encoded by the mouse *slaty* gene) also are important for melanogenesis (reviewed in Hearing, V.J., 1993, Am. J. Hum. Genet. 52:1-7). Each of the TRP proteins shares about 40% sequence identity with tyrosinase and with each other. Each of these three enzymes (tyrosinase, TRP-1 and TRP-2) is predicted to contain one transmembrane domain. Together, they form a high molecular weight complex associated with the melanosomal membrane (Orlow, S.J., *et al*., 1994, J. Invest. Dermatol. 103:196-201).

Another protein that is important for the production of melanin is the P protein. In mice, it is the product of the pink-eye dilution (p) gene. In humans, it is the product of the P gene. Humans lacking P protein function suffer from type II oculocutaneous albinism (Durham-Pierre, D., *et al*., 1994, Nature Genet. 7:176-79). *p*-null mice produce significantly less melanin than wild-type mice (Silvers, above). A wild-type human P gene, but not a mutant human P gene, can complement the hypopigmented phenotype of *p*-null mouse melanocytes (Sviderskaya, E.V., *et al*., 1997, J. Invest. Dermatol. 108:30-34). P protein is apparently needed for the production of eumelanin, but not of pheomelanin (Lamoreux, M.L., *et al*., 1995, Pigment Cell Res. 8:263-70).

The P protein is predicted to contain 12 membrane spanning domains (Gardner, J.M., *et al*., 1992, Science 257:1121-24). Consistent with this prediction, the P protein is found associated with the surface of the melanosome (Rosemblat, S., *et al*., 1994, Proc. Natl. Acad. Sci. USA 91:12071-75), which is the same membrane surface thought to be associated with the high molecular weight tyrosinase-containing complex described above.

Several authors have suggested that P protein acts as a tyrosine transporter by pumping tyrosine into the melanosome where it is converted into melanin by tyrosinase activity *(see, e.g.,* Rinchik, E.M., *et al*., 1993, Nature 361:72-76). First, the P protein bears some resemblance to transport proteins found in prokaryotes. Second, cultured *p*-null mutant mouse melanocytes, which produce much less melanin than cultured wild-type mouse melanocytes, make increased levels of melanin when high concentrations of tyrosine are added to the cells' growth medium (Sviderskaya, E.V., *et al*., above; Rosemblat, S. *et al*., 1998, Exp. Cell Res. 239:344-52). However, contradicting this suggestion, it has been found that tyrosine uptake by melanosomes is virtually the same in *p*-null and wild-type melanocytes (Gahl, W.A. *et al*., 1995, Pigment Cell Res. 8:229-233). This observation has led other authors to hypothesize that P protein is necessary for the transport into melanosomes of some other small molecule necessary for melanogenesis (summarized in Brilliant, M.H. and Barsh, G.S., 1998, above).

Other authors have speculated that P protein plays a structural role in melanosomes (Lamoreux, M.L., *et al*., above). The integrity of melanosomes is compromised in cells lacking P protein. Tyrosinase activity, and therefore melanin production, is greatly decreased in these defective melanosomes. Specifically, tyrosinase activity levels in melanocyte extracts of skin and eyes from *p*-null mice are lower than such extracts from wild-type mice (Lamoreux, M.L., *et al*., above; Chiu, E., *et al*, 1993, Exp. Eye Res. 57:301-05). Moreover, levels of tyrosinase, TRP-1 and TRP-2 proteins are lower in *p*-null tissue extracts than in wild-type extracts (Rosemblat, S., *et al*., 1998, above). Additionally, a much greater percentage of tyrosinase, TRP-1, and TRP-2 proteins are found in their monomeric forms, rather than as part of a high molecular weight complex, in *p*-null tissue extracts than in wild-type extracts (Lamoreux, M.L., *et al*., above; Chiu, E., *et al.,* above), and tyrosinase, TRP-1, and TRP-2 are all rapidly degraded in the ocular tissue of *p*-null mice (Chiu, E., *et al*., above). Finally, several authors have observed that melanosomes in *p*-null tissues and cultured melanocytes are abnormal (Russell, E.S., 1949, Genetics 34:146-66; Rosemblat, S. *et al*., 1998, above). In p-mutant melanocytes from mouse eye, very few melanosomes are observed (Orlow, S.J. and Brilliant, M.H., 1999, Exp. Eye Res. 68:147-54). In cultured mutant melanocytes, a greater than normal number of melanosomes is present, but they are smaller than those seen in wild-type melanocytes (Rosemblat, S. *et al*., 1998, above).

Thus, although P protein is known to be critical for the production of normal amounts of melanin in the skin, hair and eyes, the function of the P protein in this process has remained elusive. Instead, researchers have looked to other molecular targets for inhibition studies. For example, tyrosinase's well-characterized enzymatic activity, amenability to biochemical analysis, and pivotal role in melanogenesis have made it an inviting target for inhibition studies *(see, e.g.,* Tasaka, K., *et al*., 1998, Meth. Find. Exp. Clin. Pharmacol. 20:99-109; lida, K., *et al*., 1995, Planta Med. 61:425-28; Reish, O., *et al*., 1995, Am. J. Hum. Genet. 57:127-32; Shirota, S., *et al*., 1994, Biol. Pharm. Bull. 17:266-69; Kameyama, K., *et al*., 1989, Differentiation 42:28-36). Researchers have also focused on the effects of intercellular signaling molecules on melanogenesis *(see, e.g.,* Furumura, M. *et al*., 1998, Proc. Natl. Acad. Sci. USA 95:7374-78; Sakai, C., *et al*., 1997, EMBO J. 16:3544-52; McLeod, S.D. *et al*., 1995, J. Endocrinol. 146:439-47).

### 3.. Summary of the Invention

The present invention provides novel screens for the identification of compounds that inhibit or increase melanogenesis in melanogenic cells. The development of these assays is based, in part, on the discovery that some compounds that inhibit melanogenesis do so by causing a mislocalization of tyrosinase, the key enzyme in melanin synthesis.

The P protein is a pivotal target for compounds and drugs to decrease or increase pigmentation of the skin, hair and/or eyes. Accordingly, in one aspect, the present invention provides, for the first time, screens for compounds that inhibit or enhance P protein function based, in part, on the discovery that P protein function is required for proper cellular localization of tyrosinase and other melanosomal proteins and is required for both full tyrosinase activity and melanogenesis in melanogenic cell types such as, for example, melanocytes and melanoma cells.

Wild-type melanogenic cells target tyrosinase primarily to melanosomes. Some tyrosinase is also secreted by these cells. P protein-compromised melanogenic cells mislocalize tyrosinase. They secrete significantly more tyrosinase than wild-type melanogenic cells, and also contain tyrosinase in non-melanosomal vesicles. Tyrosinase that is secreted from melanogenic cells, regardless of whether the cells have normal or inhibited P protein function, is enzymatically active in the growth or incubation medium, where it can convert tyrosine into melanin.

In one aspect, the present invention provides a method of screening for compounds that inhibit melanogenesis in melanogenic cells, comprising incubating these cells in medium containing a compound to be tested, and identifying compounds that cause a change in the cellular localization of tyrosinase in these cells. Mislocalization of tyrosinase can indicate inhibition of melanogenesis.

In a still further aspect, the present invention provides methods of screening for compounds that increase melanogenesis in melanogenic cells, comprising incubating melanogenic cells in medium containing a compound to be tested, and identifying compounds that cause a decrease in the amount of tyrosinase secreted by the cells relative to the amount of tyrosinase retained by the cell, wherein such relative decrease in the amount of tyrosinase secreted indicates that the compound is a candidate for a compound that increases melanogenesis.

Another aspect of the invention is based, in part, on the discovery that non-melanogenic cells can be made to produce active tyrosinase by transfecting them with a heterologous tyrosinase-encoding gene. The tyrosinase activity of these cells is dramatically increased by cotransfection with a heterologous P protein-encoding gene. Maximal tyrosinase activity in these cells is therefore dependent upon P protein function. When cells expressing both heterologous tyrosinase and heterologous P protein are treated with drugs that inhibit P protein function such as, for example, imipramine, the tyrosinase activity of these cells is reduced to that of cells expressing heterologous tyrosinase alone. Imipramine and other drugs that inhibit P protein function do not otherwise affect tyrosinase activity in cells that express heterologous tyrosinase but that do not express heterologous P protein.

Accordingly, in a further aspect, the present invention provides methods of screening for compounds that affect (*e*.*g*., either inhibit or increase) P protein function in cells that do not ordinarily express tyrosinase and/or P protein, comprising manipulating these cells so that they express both tyrosinase and P protein, and treating the cells with a compound to be tested. The tyrosinase activity of these cells is measured. Compounds that affect (e.g., inhibit or increase) the tyrosinase activity of these cells, but that do not affect the tyrosinase activity of cells expressing tyrosinase alone, are identified as compounds that affect tyrosinase in a P protein dependent manner.

In a further aspect, the present invention provides methods for modeling chemical compounds known to affect or mimic the function of P protein. Analogs of the modeled compound are selected or designed, and screened for the ability to affect P protein function. By using analogs of a compound known to affect or mimic P protein function, new and better compounds that affect or mimic P protein function can be discovered using the methods of the present invention.

In a still further aspect, the present invention provides methods for using, in medicinal and cosmetic compositions, compounds that affect or mimic the function of P protein, thereby treating a disease, condition, or disorder involving the production (underproduction or overproduction) of melanin.

### 4. Brief Description of the Figures

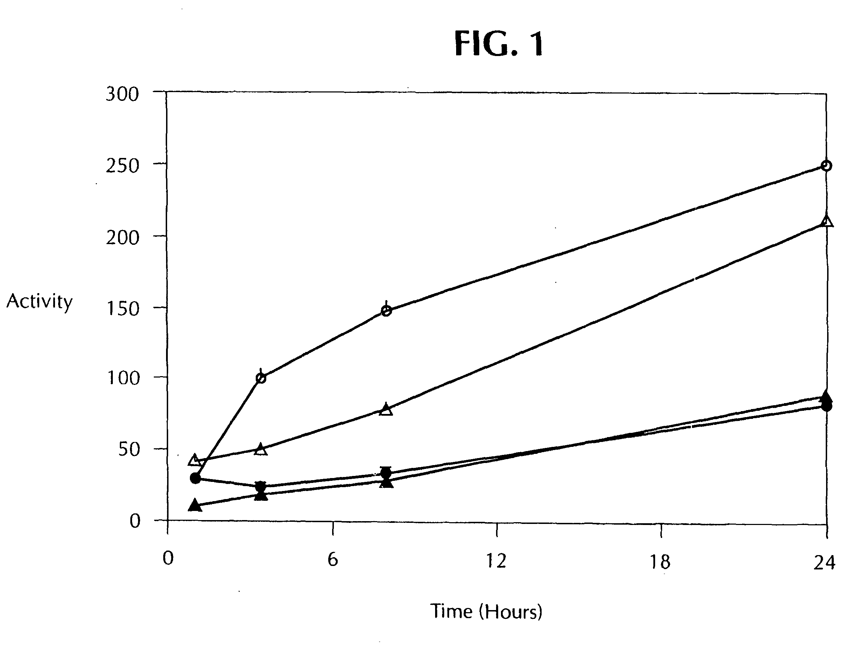

FIG. 1. Tyrosinase activity in media from cultured melanocytes. Melan-a melanocytes cultured from black mice, and melan-p melanocytes cultured from mice lacking a P gene transcript, were separately cultured in DMEM containing low (0.03 mM) or high (0.3 mM) tyrosine for the indicated time period. The activity of tyrosinase was determined at specific time intervals in media from melanocytes. The medium was dialyzed prior to determining the enzyme activity, which is expressed as cpm of tritiated water generated per hour. ▲ Melan-a, high tyrosine; • melan-a, low tyrosine; Δ melan-p1, high tyrosine; ° melan-p1, low tyrosine. Increasing tyrosinase activity in the media removed from melan-p cells, which have no P protein transcripts, grown in the presence of 0.03 mM tyrosine, reflects an increased secretion of tyrosinase by these cells. In contrast, melan-a cells, which represent wild-type melanocytes, secrete significantly less tyrosinase into the media. Growing melan-p cells in the presence of high tyrosine partially alleviated the P-deficient phenotype.

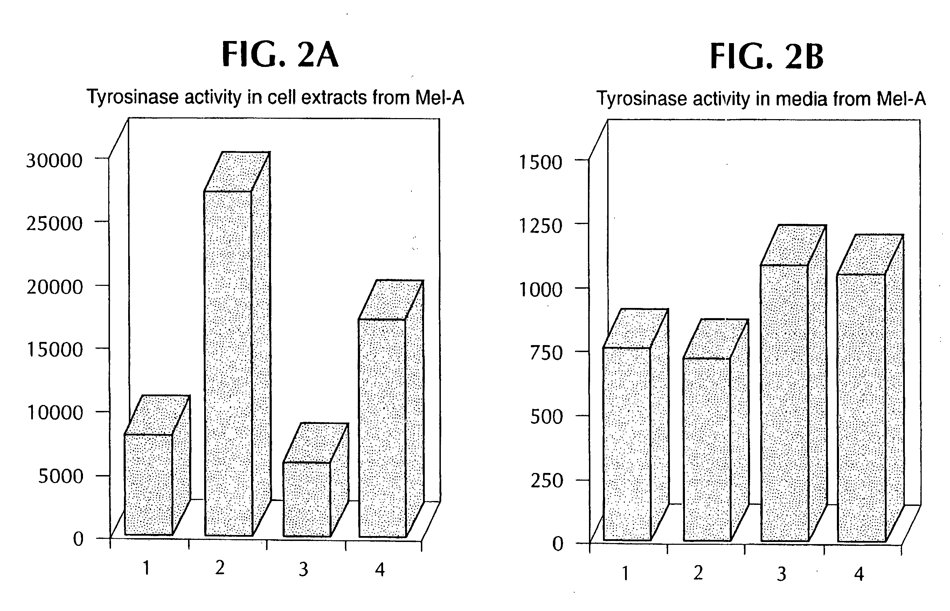

FIG. 2. Tyrosinase activity in cell extracts and media from melan-A cells. Cultured melan-a melanocytes were incubated for 48 hours in the presence of benztropine, imipramine, nitroquipazine, or left untreated. Incubation media or cell extracts were assayed for tyrosine hydroxylase activity, as in FIG. 1. Column 1, untreated melanocytes; Column 2, melanocytes treated with benztropine; Column 3, melanocytes treated with 10,11-Dihydro-n,n-dimethyl-5H-dibenz[b,f]azepine-5-propanamine (imipramine); Column 4, melanocytes treated with 6-Nitro-2-(1-piperazinyl)-quinoline maleate (nitroquipazine). In FIG. 2a (left), tyrosine hydroxylase activity of melan-a cell extracts is measured in cpm [³H]H₂O/60 micrograms protein/hr. In FIG. 2b (right), tyrosine hydroxylase activity in media from melan-a cells is measured in cpm [ ³H]H₂O/hr normalized to the amount of cell extract protein. The tyrosine hydroxylase activity of extracts from melan-a cells incubated with benztropine (column 2 in FIG. 2a.) and nitroquipazine (column 4 in FIG. 2a) is higher than that seen in untreated cells. The extracts from cells treated with imipramine (column 3 in FIG. 2a) show a reduced activity. The effects of the drugs on the enzyme activity of the cell extracts is not reflected in the activity of the enzyme secreted into the media. While benztropine has little effect on activity (column 2 in FIG. 2b), imipramine (column 3 in FIG. 2b) and nitroquipazine (column 4 in FIG. 2b) cause a significant increase in activity in the media.

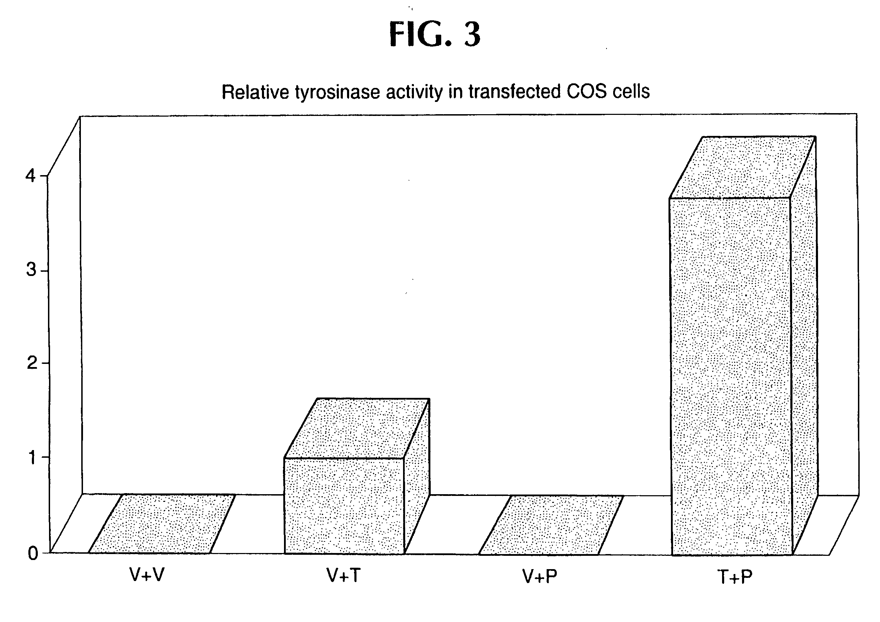

FIG. 3. Relative tyrosinase activity in transfected COS cells. COS cells were transfected with two doses of the vector alone (V+V), one dose of the vector alone and one dose of the vector carrying a tyrosinase-encoding gene (V+T), one dose of the vector alone and one dose of the vector carrying a P protein-encoding gene (V+P), or one dose each of the vectors carrying a tyrosinase-encoding gene and a P protein-encoding gene (T+P). Equal quantities of cell extract protein were assayed for tyrosine hydroxylase activity. Relative activities shown are calculated as the activity of the test sample divided by the activity of the V+T sample. The introduction of an expression plasmid carrying the tyrosinase gene (V + T) results in tyrosine hydroxylase activity in COS cells. This activity is the direct result of the tyrosinase-encoding plasmid, since transfection with the expression vector (V + V) alone does not generate any tyrosine hydroxylase activity. The tyrosine hydroxylase activity in cells carrying the tyrosinase-encoding plasmid can be increased almost 4-fold by co-transfection with the P gene expression plasmid (T + P). This increase is the result of an interaction between tyrosinase and P protein, since the introduction of P (V + P) without tyrosinase generates no tyrosine hydroxylase activity.

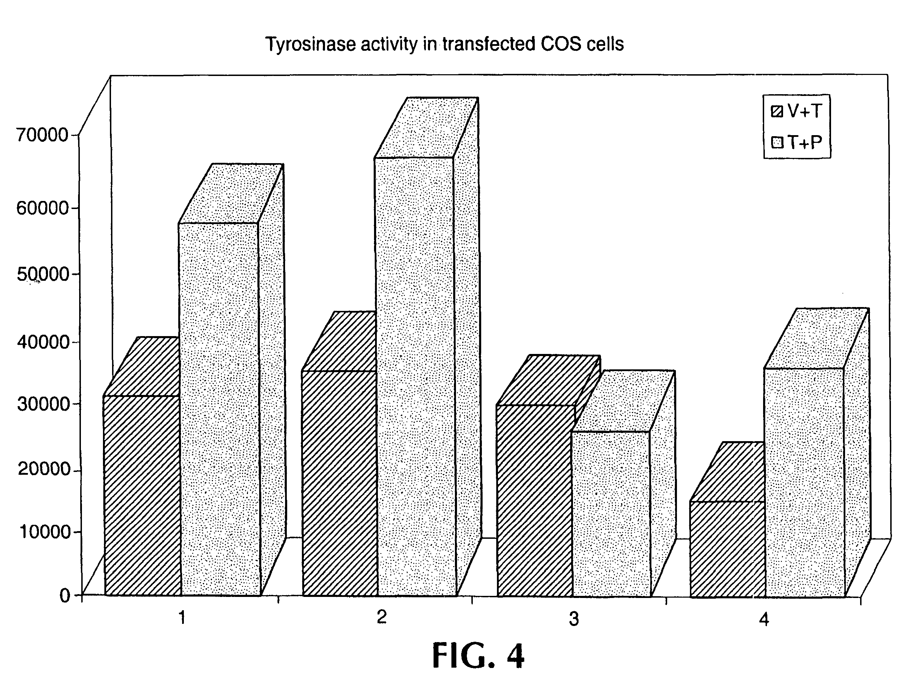

FIG. 4. Tyrosinase activity in transfected COS cells. COS cells transfected with a vector carrying a tyrosinase-encoding gene, or with a first vector carrying a tyrosinase-encoding gene and with a second vector carrying a P protein-encoding gene as in FIG. 3, were treated with benztropine, imipramine, nitroquipazine, or left untreated, as in FIG. 2. Cell extracts were prepared as in FIG. 3 The tyrosine hydroxylase activity of cell extracts was determined as in FIG. 1 as a measure of tyrosinase activity. Column 1, untreated transfectants; Column 2, transfectants treated with benztropine; Column 3, transfectants treated with imipramine; Column 4, transfectants treated with nitroquipazine. Tyrosine hydroxylase activity is measured in cpm [ ³H]H₂O/60 micrograms protein/hr. Cells cotransfected with a tyrosinase-encoding gene and a P protein-encoding gene (T + P) show a higher tyrosine hydroxylase activity than cells transfected with a tyrosinase-encoding gene alone (V + T) (column 1). This effect is not altered by incubation of cells in the presence of benztropine (column 2) or nitroquipazine (column 4). The presence of imipramine, however, abolishes the effect of P protein while appearing to have little effect on the activity in the cells with tyrosinase alone (column 3).

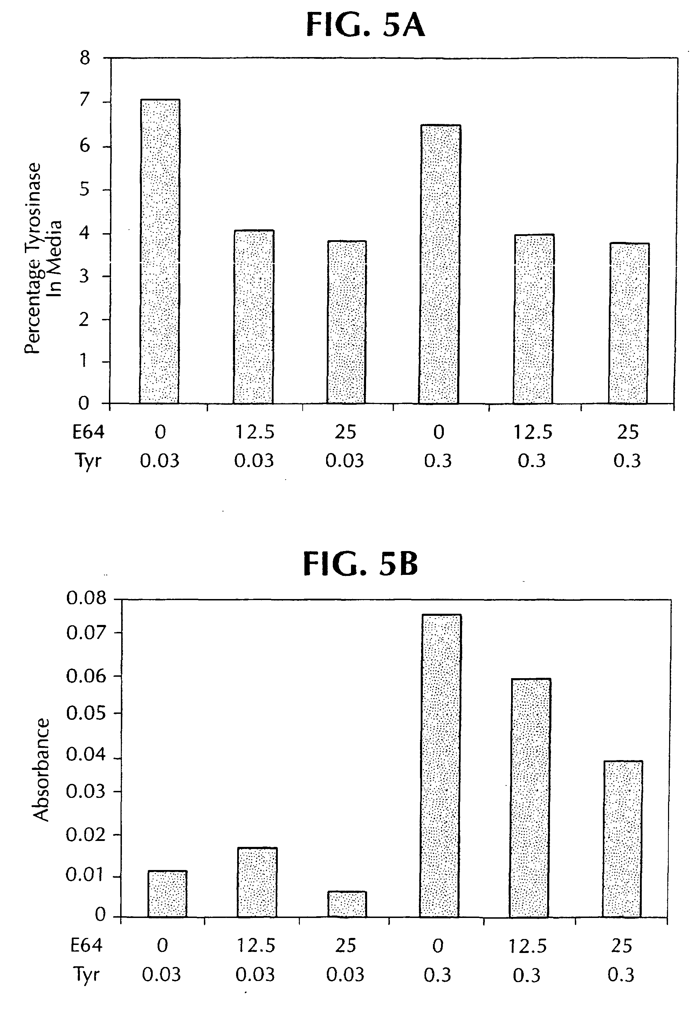

FIG. 5. Levels of secreted tyrosinase are elevated in melan-p1 and are reduced by inhibition of cysteinyl proteases. (a) Melan-p1 cells incubated in low (0.03 mM) tyrosine and high (0.3 mM) tyrosine (TYR) were treated for 48 hours with increasing concentrations of the protease inhibitor E64 (µM). The tyrosinase activity in the media is expressed as a percentage of total activity in the extract and medium. (b) The concentration of melanin was determined by solubilizing the cell pellet and measuring the absorbance at 470 nm.

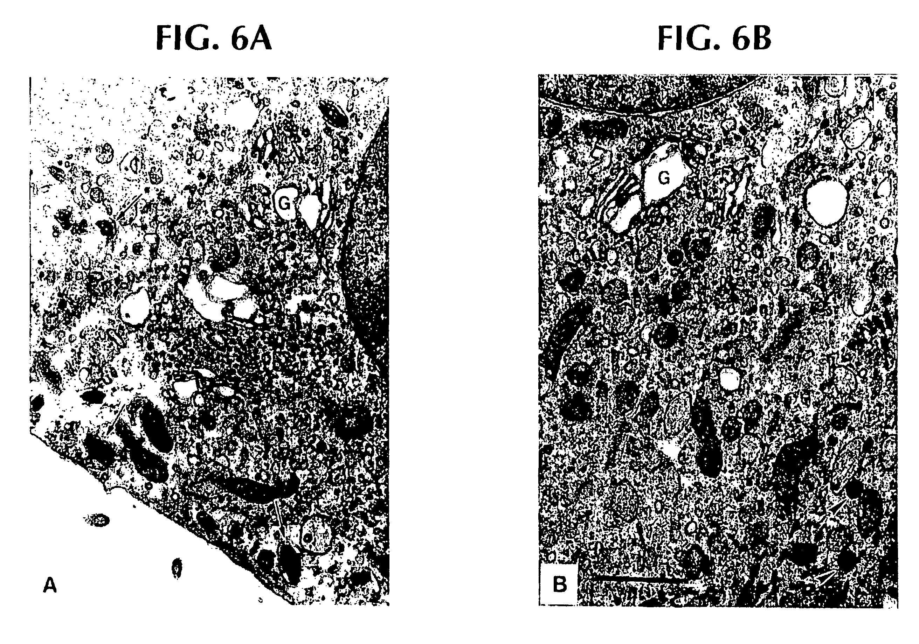

FIG. 6. Ultrastructure of cultured melanocytes. The peri-nuclear area of melan-a (A) and melan-p1 (B) melanocytes demonstrating the Golgi apparatus (G). Melanosomes in the melan-a cell are of stage I, II, III, and predominantly stage IV (arrows). Melanosomes in melan-p1 cells are predominantly stage I and II with an occasional early stage III (arrowheads); no stage IV melanosomes were observed. BAR = 1.0 micron.

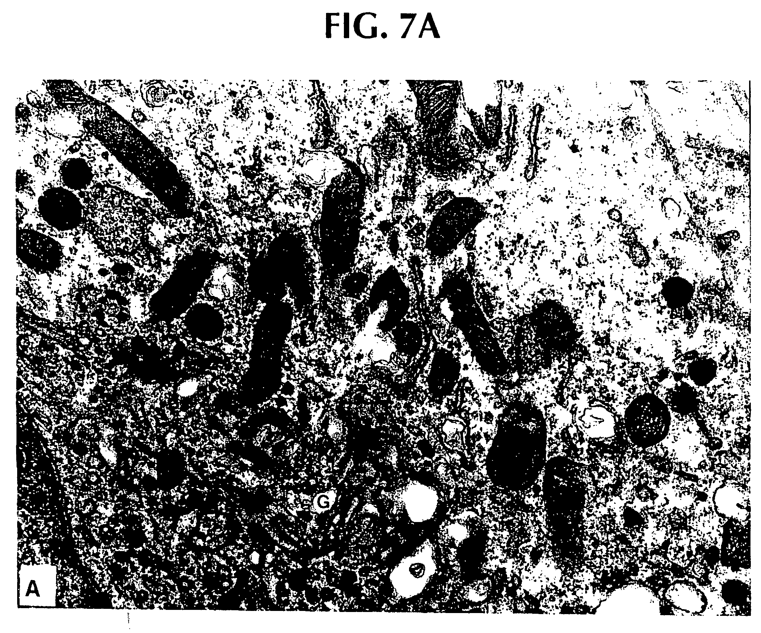

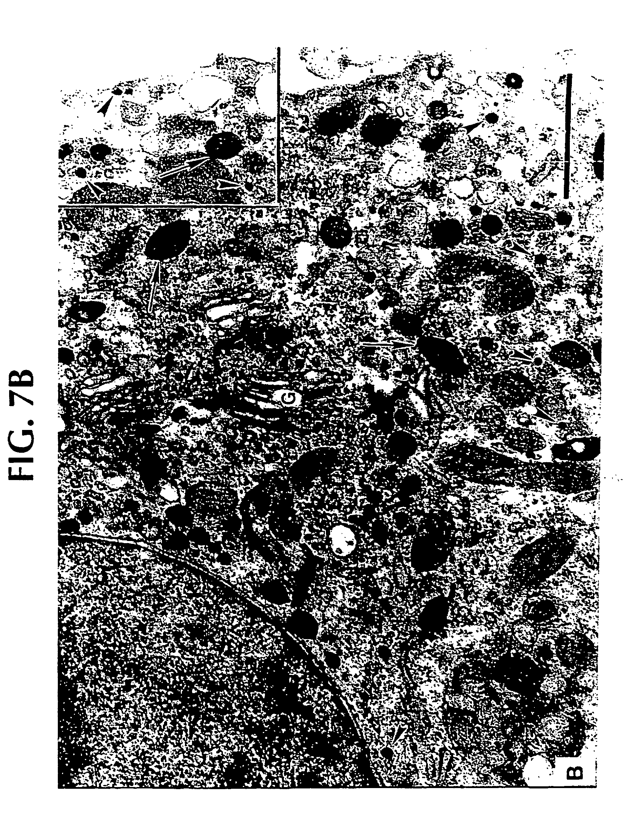

FIG. 7. Ultrastructure of cultured melanocytes processed for DOPA histochemistry. The perinuclear area of melan-a (A) and melan-p1 (B) melanocytes demonstrating the Golgi apparatus with DOPA reaction product in the cisternae and 50 nm vesicles of the TGN (G). The 50 nm vesicles are confined to the TGN in the melan-a cells and radiate away from the TGN in melan-p1 cells (arrowheads), and can be observed in close proximity to the plasma membrane (inset). Occasional stage III melanosomes are noted (arrows). BAR = 1.0 micron.

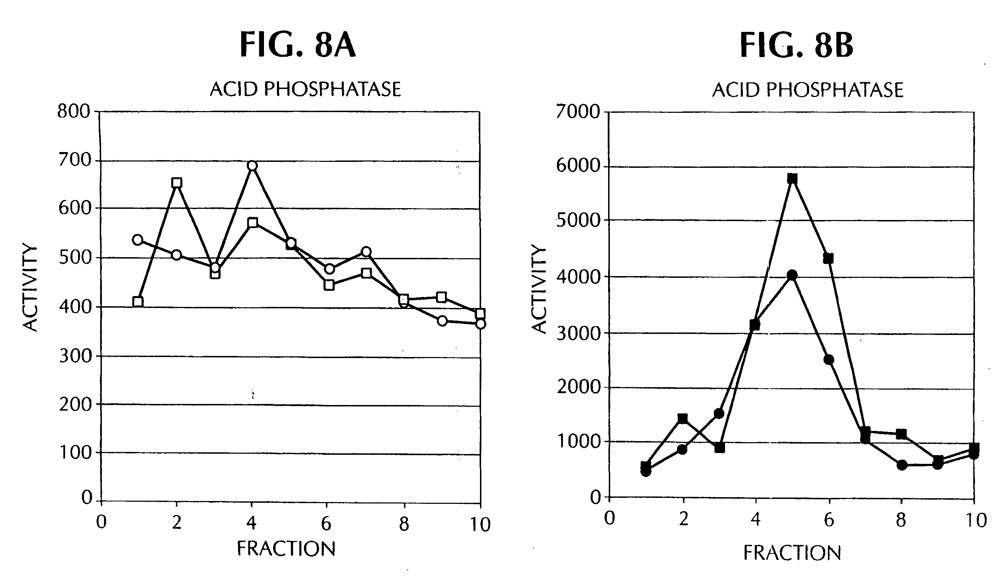

FIG. 8. Acid phosphatase targeting in melan-A and melan-P cells. Acid phosphatase activity was measured in fractionated membranes from melan-a (squares) and melan-p cells (circles) as described below in the Examples (Section 10). FIG. 8A = small granule fractions. FIG. 8B = large granule fractions.

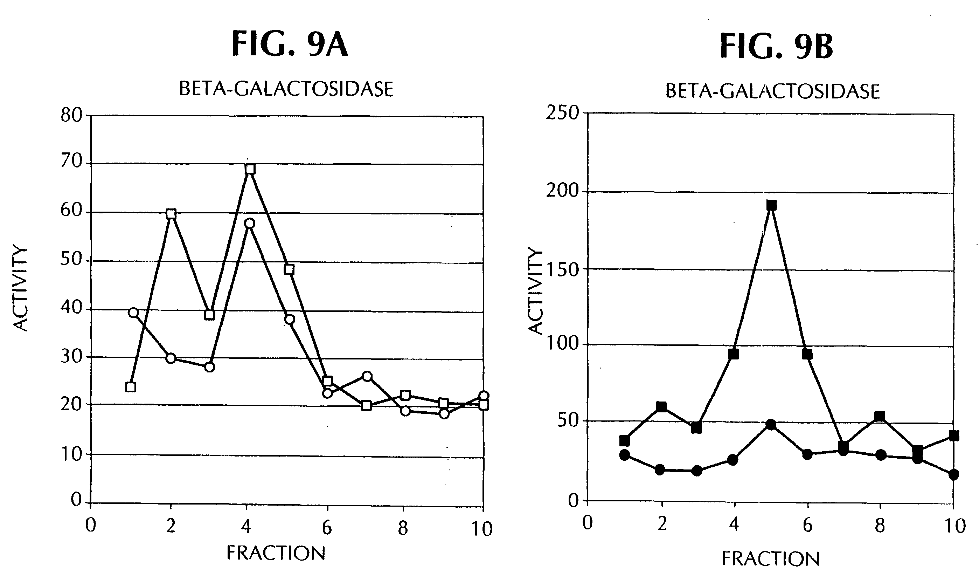

FIG. 9. β-galactosidase targeting in melan-A and melan-P cells. β-galactosidase activity was measured in fractionated membranes from melan-a (squares) and melan-p cells (circles) as described below in the Examples (Section 10). FIG. 9A = small granule fractions. FIG. 9B = large granule fractions.

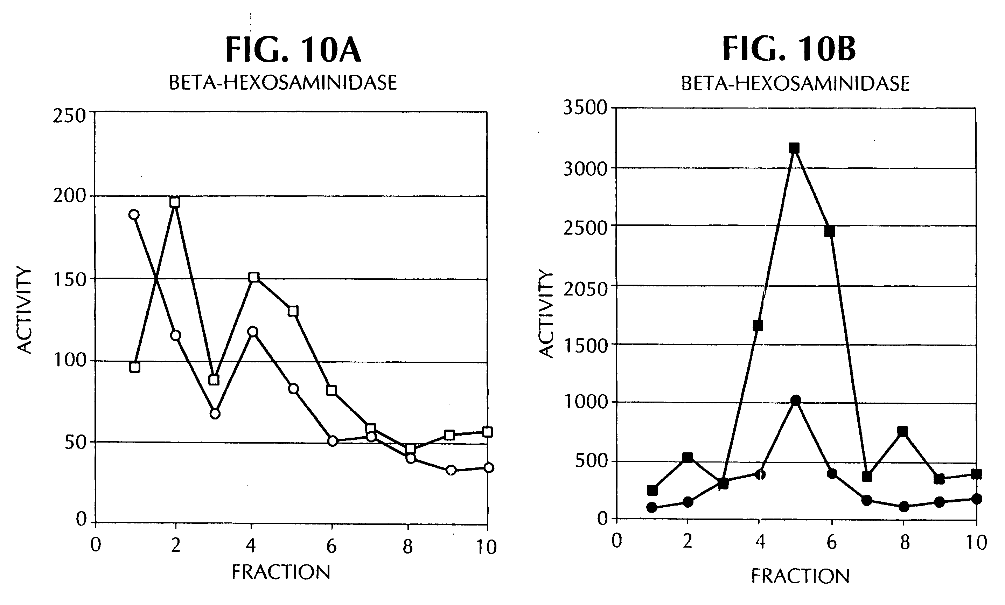

FIG. 10. β-hexosaminidase targeting in melan-A and melan-P cells. β-hexosaminidase activity was measured in fractionated membranes from melan-a (squares) and melan-p cells (circles) as described below in the Examples (Section 10). FIG. 10A = small granule fractions. FIG. 10B = large granule fractions.

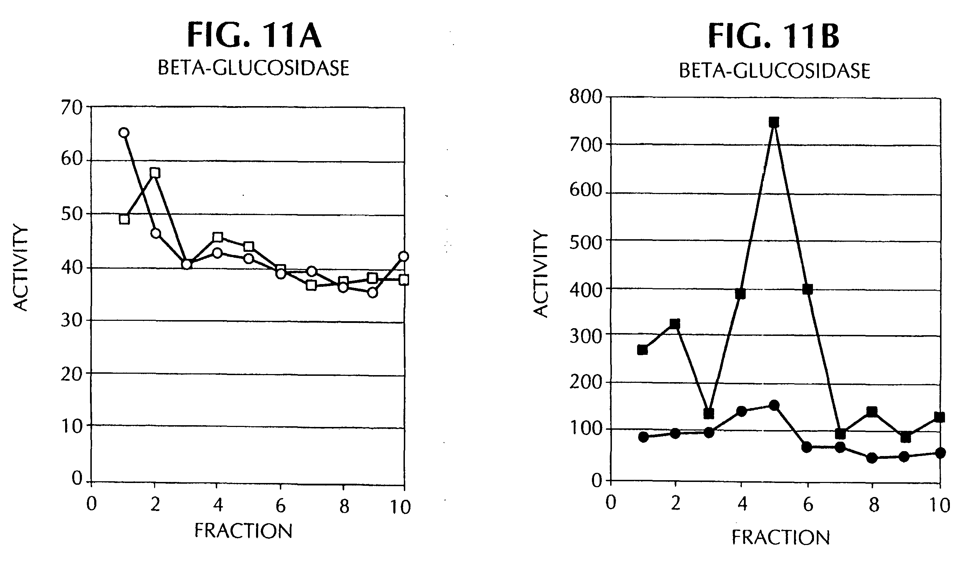

FIG. 11. β-glucosidase targeting in melan-A and melan-P cells. β-glucosidase activity was measured in fractionated membranes from melan-a (squares) and melan-p cells (circles) as described below in the Examples (Section 10). FIG. 11A = small granule fractions. FIG. 11B = large granule fractions.

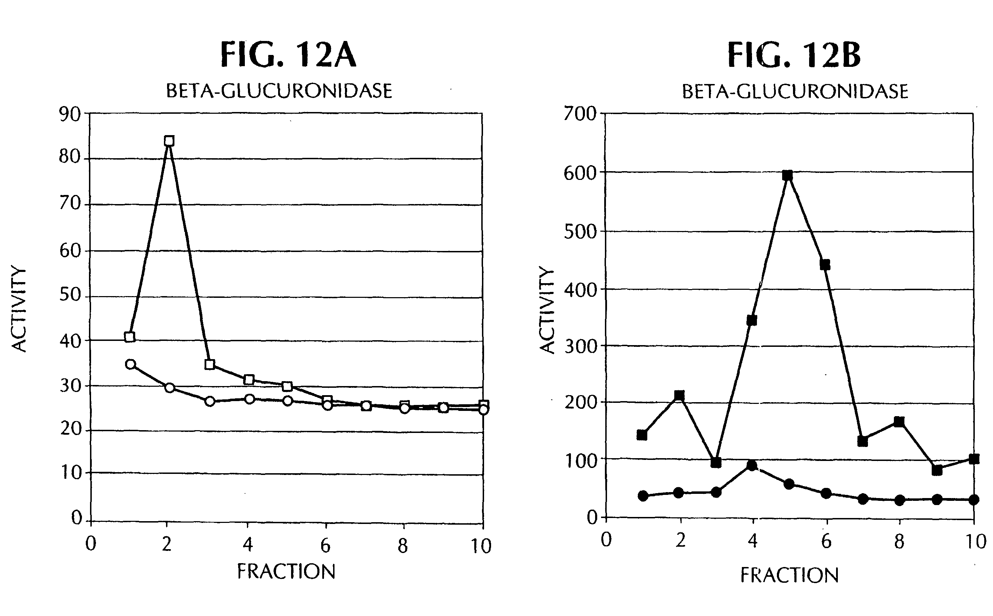

FIG. 12. β-glucuronidase targeting in melan-A and melan-P cells. β-glucuronidase activity was measured in fractionated membranes from melan-a (squares) and melan-p cells (circles) as described below in the Examples (Section 10). FIG. 12A = small granule fractions. FIG. 12B = large granule fractions.

### 5. Detailed Description of the Invention

The invention is based, in part, on the discovery that compounds that cause melanogenic cells to mislocalize tyrosinase (*e.g.*, to increase the amount of tyrosinase secreted or the amount of tyrosinase found in non-melanosomal vesicles) also inhibit melanogenesis. For purposes of the present invention, the term "melanogenic cells" is defined as cells that contain pigmented melanosomes (*e.g.*, melanocyte cells and melanoma cells). Melanogenic cells can include, for example, melanogenic cells that express heterologous melanosomal proteins. For example, in preferred embodiments, the coding sequence or sequences of the P protein gene, tyrosinase gene, TRP-1 gene, and/or TRP-2 gene in a mouse melanogenic cell can be mutated or deleted, and the cell engineered to express instead the corresponding coding sequence of the human P protein gene, tyrosinase gene, TRP-1 gene, and/or TRP-2 gene.

Another aspect of the present invention is based, in part, on the discovery that the P protein is necessary to correctly localize tyrosinase predominantly to the membrane of melanosomes.

Yet another aspect of the present invention is based on the finding that melanocytes treated with compounds that inhibit P protein function accumulate reduced amounts of intracellular melanin, and secrete increased amounts of tyrosinase into the growth medium.

Still another aspect of the present invention relates to the discovery that, in the presence of the P protein, the enzymatic activity of tyrosinase protein in cultured cells is augmented.

Accordingly, the present invention provides novel methods of screening for compounds that inhibit melanogenesis. Compounds identified using the methods of the present invention are useful for treating diseases and cosmetic defects associated with the underproduction or overproduction of melanin.

In another aspect, the present invention relates to the discoveries that wild-type melanogenic cells with normal P protein function secrete some tyrosinase, and that compounds that increase secretion of tyrosinase in a P protein dependent manner also inhibit melanogenesis. Accordingly, the present invention provides novel methods of screening for compounds that increase melanogenesis by increasing the function of P protein. For purposes of this application, compounds that increase the function of P protein and compounds that decrease the function of P protein are collectively referred to herein as "compounds that affect the function of P protein." Still another aspect of the invention is a method of screening for compounds that increase melanogenesis by mimicking the function of P protein. For purposes of the invention, "compounds that mimic the function of P protein" are compounds that are not P proteins, yet when they are administered to, or incubated with, melanogenic cells that do not contain P protein, they serve to restore at least in part the correct targeting of tyrosinase to the melanogenic membrane. Melanogenic cells that do not contain P protein may be cells that do not express P protein transcripts (such as melan-p cells, described herein) or melanogenic cells that do not express a functional P protein gene product.

### 5.1 Methods of Screening for Inhibitors or Inducers of Melanogenesis

### 5.1.1 Methods of Screening for Inhibitors of Melanogenesis Using Melanogenic Cells

In order for melanogenic cells to engage in robust melanogenesis, they must target their tyrosinase predominantly to the melanosomal membrane. Consequently, in one aspect, the methods of the present invention entail screening for compounds that cause melanogenic cells to mislocalize tyrosinase. P protein function is necessary for the correct cellular localization of tyrosinase. Therefore, in another aspect, the methods of the present invention entail screening for compounds that inhibit P protein function, thereby causing melanogenic cells to mislocalize tyrosinase. Such methods are based, in part, on the discovery that cultured melanocytes that have been genetically altered to eliminate P protein function secrete significantly more tyrosinase into the growth medium than wild-type melanocytes. Compounds, such as, *e*.*g*., imipramine, that reduce or eliminate P protein function will have the same effect. Thus, the cellular mislocalization of tyrosinase by cells treated with a test compound indicates that the test compound inhibits melanogenesis. Mislocalization of tyrosinase resulting in secretion can be detected initially by assaying either the level of tyrosinase activity in the medium or cells, or the level of tyrosinase protein in the medium or cells. Test compounds that cause an increase in secretion of tyrosinase, or a decrease in intracellular tyrosinase, are candidates for compounds that inhibit melanogenesis by inhibiting P protein function. Such candidate compounds can be further investigated for their effect on melanogenesis, and/or for their effects in both the presence and absence of P protein, as described more fully below. If the effect of the candidate compound depends upon the presence of P protein, then the compound inhibits the function of P protein.

Because growing P-protein-deficient melanocytes in the presence of high levels of tyrosinase in the medium can partially rescue the P-protein-deficiency, it is preferable, but not necessary, that a screen for inhibitors of melanogenesis is carried out in the presence of low amounts of tyrosine in the media, *e.g.,* 0.01-0.05 mM tyrosine, more preferably 0.014-0.03 mM tyrosine.

### 5.1.1.1 Methods of Screening for inhibitors of Melanogenesis Using Assays for Tyrosinase activity

Wild-type melanogenic cells grown in *in vitro* culture will synthesize melanin inside of melanosomes as they do *in vivo.* In these cultured cells, tyrosinase is found predominantly in the melanosomal membrane, although some tyrosinase is also secreted. The tyrosinase that is found in the melanosomal membrane is held in place by a C-terminal transmembrane domain and has its active site disposed toward the melanosomal lumen. By contrast, in melanogenic cells inhibited for melanogenesis through either a mutation in P protein or a compound that inhibits P protein function, tyrosinase will be mislocalized. A significantly greater fraction of the cells' tyrosinase is secreted from the cells into the growth or incubation medium. Additionally, the secreted tyrosinase polypeptide will be shorter than that found in wild-type cells because it lacks its C-terminal membrane anchor. The secreted tyrosinase, however, is enzymatically active in the growth or incubation medium where it can synthesize melanin from extracellular tyrosine. Consequently, tyrosine-containing growth or incubation media from melanogenic cells that have been inhibited for melanogenesis will turn dark. The higher the concentration of tyrosine in the medium, the darker the medium becomes, and the higher the concentration of tyrosinase in the medium, the faster the medium darkens. Because melanogenic cells that are not inhibited for melanogenesis secrete significantly less tyrosinase, the tyrosine-containing growth or incubation media in which they are cultured will not become as dark.

This discovery can be used in a novel method of screening to identify compounds that inhibit or modulate melanogenesis. Melanogenic cells are grown in culture or incubated in medium containing tyrosine. The cells are treated with a test compound. If the test compound causes tyrosinase to be mislocalized and secreted from the treated cells, then tyrosine in the medium will be converted into melanin, darkening the medium. An assay is used wherein the color of the medium is compared to the color of the medium of the melanogenic cells grown or incubated under similar conditions but without the test compound (a control medium). If the medium of the cells treated with the test compound turns darker than the control medium, then the test compound is identified as candidate for a compound that inhibits melanogenesis.

More typically, in order to obtain at least semi-quantitative data, the media from the cells is first filtered, centrifuged and/or dialyzed prior to assay for tyrosinase activity. These types of treatments remove potentially confounding factors such as cells or particulate matter (*e.g.*, melanosome or shed membranes) containing tyrosinase that could compete for substrate, and/or remove excess free tyrosine that might compete with labeled substrate. Any of a number of alternative ways of measuring tyrosinase can be carried out, such as by using any of the enzymatic tyrosinase activities including but not limited to converting tyrosine to dihydroxyphenylalanine (DOPA), DOPA to dopaquinone, and oxidation of 5,6-dihydroxyindole to 5,6-indolequinone. For example, when assaying for the tyrosine hydroxylase activity of tyrosinase, non-tyrosine or altered tyrosine substrates of tyrosinase can be used in addition to tyrosine. In one aspect, melanogenic cells are grown or incubated in culture with a test compound. After pretreatment of the medium, a non-tyrosine or altered tyrosine substrate of tyrosinase is added to the growth or incubation medium. The substrate can be a homolog, analog, or derivative of tyrosine which can be a natural product or produced synthetically. Tyrosinase activity in the medium converts the substrate into its product.

An assay is then used to detect the presence of the product and/or the absence of the substrate. One non-limiting assay is a colorimetric assay. In methods of screening for compounds that inhibit melanogenesis that use a colorimetric assay, a substrate is chosen that changes color when it is acted upon by tyrosinase. That is, the wavelength of light absorbed by the substrate is different than the wavelength of light absorbed by the products of the reaction catalyzed by tyrosinase. The wavelength of light absorbed by the substrate and/or by the products can be in the visible light range, the infrared range, or the ultraviolet range of the spectrum. The concentration of substrate, incubation time, and other reaction conditions can be chosen such that the speed and/or intensity of the color change is proportional to the amount of tyrosinase activity in the cells' growth or incubation medium. The color change can be detected by direct observation, or measured by a device, such as a spectrophotometer and compared, *e.g.*, to a standard curve prepared using varying amounts of product.

An example of a colorimetric assay is the DOPA oxidase assay. In one method of screening for compounds that inhibit melanogenesis using this assay, a compound to be tested for its ability to inhibit melanogenesis is added to the growth or incubation medium of melanogenic cells. After filtration, centrifugation and/or dialyzation of the medium, L-DOPA is added under conditions that would otherwise allow for tyrosinase to catalyze the formation of dopachrome from L-DOPA. In a preferred though non-limiting embodiment, the final concentration of L-DOPA in the medium is about 5 x 10⁻³ M, the pH is about 7.4, and the temperature is about 25°C. Increased absorbance of the medium at 475 nm (relative to the absorbance at 475 nm of medium from similar cells grown under similar conditions but without the test compound) indicates the formation of dopachrome by tyrosinase in the medium, and therefore the inhibition of melanogenesis by the test compound.

Alternatively, as dopachrome absorbs light within the visible range, the presence of dopachrome, and hence the inhibition of melanogenesis, can be determined by direct inspection of the reaction, without the aid of a spectrophotometer.

Another assay is a radiometric assay. In an alternative method of screening for compounds that inhibit melanogenesis using this assay, substrate is radioactively labeled and added to the growth or incubation medium to be assayed. If tyrosinase is present in the medium, it cleaves the substrate into a labeled product and an unlabeled product. The amount of radioactive substrate that has been converted into radioactive product is measured. The concentration of substrate, time of incubation, temperature of incubation, and other reaction conditions can be chosen so that the amount of radioactive product produced is proportional to the amount of tyrosinase in the growth or incubation medium being assayed. A greater amount of labeled product in the medium from cells treated with the test compound than in the medium of similar cells grown under similar conditions but without the test compound indicates that the test compound is a candidate for a compound that inhibits melanogenesis.

An example of this type of assay is the radiometric tyrosine hydroxylase assay. In this assay, the amount of [³H]H₂O released from [³H]tyrosine as a result of the tyrosine hydroxylase activity of the tyrosinase enzyme is measured. In one method of screening for compounds that inhibit melanogenesis that uses this assay, media from melanogenic cells is harvested and cells removed. Additionally, the media can be dialyzed before assay. For assays, 1.5 microCi [³H]tyrosine is added to the media and incubated for defined lengths of time at appropriate temperature for enzyme activity. Unreacted [³H]tyrosine is removed from the medium by adsorption onto 10% (w/v) activated charcoal in 0.1 M citric acid, then treated with 50% (w/v) Dowex resin solution. The medium is mixed with scintillant and counted in a beta-counter. A significant increase in [³H]H₂O levels in the medium of cells that were treated with a test compound compared to [³H]H₂O levels in the medium of similar cells grown under similar conditions without test compound indicates that the test compound is a candidate for a compound that inhibits melanogenesis.

Yet another example of this type of assay is the radiometric melanin synthesis assay. In this assay, the amount of [¹⁴C]tyrosine or [¹⁴C]DOPA incorporated into [¹⁴C]melanin is measured. In a non-limiting example of a method of screening for compounds that inhibit melanogenesis that uses this assay, melanogenic cells are grown or incubated in medium containing a test compound. The medium is harvested and 1 microCi [¹⁴C]tyrosine is added and incubated at the appropriate temperature for four hours. The reaction is terminated with ice-cold 10% (w/v) TCA and the mixture vortexed and frozen for 24 hours. The mixture is then thawed and centrifuged at 1000 g for 15 minutes at 4°C. The pellet is resuspended in ice-cold 5% TCA (w/v). This step is repeated twice. The final pellet containing [¹⁴C]melanin is solubilized in Soluene®-350 (Packard Instrument Company, Meriden, CT) for four hours, mixed with scintillant, and counted. Alternatively, the pellet can be collected on filter paper and counted. A significant increase in [¹⁴C]melanin levels in media of cells that were treated with a test compound compared to [¹⁴C]melanin levels in media of similar cells grown under similar conditions but without the test compound indicates that the test compound is a candidate for a compound that inhibits melanogenesis.

Another assay is a fluorescence assay. In this assay, the substrate and/or its products are fluorescent. The wavelength of light absorbed and/or emitted by the substrate is different from the wavelength of light absorbed and/or emitted by the products. In a non-limiting example of a method of screening for compounds that inhibit melanogenesis that uses this assay, melanogenic cells are grown in culture in the presence or absence of the test compound. After a period of growth or incubation, the media is removed, tyrosinase substrate added, and a fluorometer used to measure the fluorescence of the growth or incubation medium. The concentration of substrate, time of incubation, temperature of incubation and other reaction conditions can be chosen so that the change in fluorescence is proportional to the levels of tyrosinase activity in the medium being analyzed. A significant difference in fluorescence levels of media from cells treated with a test compound compared to fluorescence levels of media from similar cells grown under similar conditions but without the test compound, indicates that the test compound is a candidate for a compound that inhibits melanogenesis.

Another type of assay involves the precipitation of reaction products. In an example of a method of screening for compounds that inhibit melanogenesis that uses this assay, a substrate of tyrosinase is incubated with the harvested growth or incubation medium under conditions that promote the activity of tyrosinase. The substrate is acted upon by tyrosinase to produce a reaction product that can be precipitated. The reaction product is precipitated. The reaction product can be precipitated from the medium by, for example, increasing or decreasing the temperature of the medium, increasing or decreasing the pH of the medium, increasing or decreasing the ionic strength or salt concentration of the medium, or otherwise altering the medium appropriately, or by centrifugation if the reaction product is insoluble. Substrate concentrations, time of incubation, temperature of incubation, and other reaction conditions can be chosen so that the amount of precipitable reaction product is proportional to the levels of tyrosinase activity in the medium being analyzed. A significant increase in the amount of reaction product precipitated from the media of cells treated with a test compound compared to the amount of reaction product precipitated from the media of similar cells grown under similar conditions but without the test compound indicates that the test compound is a candidate for a compound that inhibits melanogenesis.

### 5.1.1.2 Methods of Screening for Inhibitors of Melanogenesis Using Assays for Tyrosinase Protein

The preceding methods of screening serve to identify inhibitors of melanogenesis using assays of tyrosinase activity (*i*.*e*., the protein's enzymatic activities). The present invention further provides a method of screening inhibitors of melanogenesis using assays for either extracellular or intracellular tyrosinase protein levels. As explained above, tyrosinase is primarily localized to the melanosomal membrane in melanogenic cells. Compounds that cause tyrosinase to be mislocalized serve to inhibit melanogenesis. In the following methods of screening, assays for determining tyrosinase protein levels and/or locations are used. This can be done using any of the standard techniques of protein detection known in the art. The protein detection assays employed herein can be those described in Harlow and Lane (Harlow, E. and Lane, D., 1988, *Antitiodies: A Laboratory Manual*, Cold Spring Harbor Laboratory Press, Cold Spring Harbor, New York), which is incorporated herein by reference in its entirety. These assays include, but are not limited to, immunological assays, including Western blots, solid-phase radioimmunoassays, *in situ* hybridizations, and immunoprecipitations. Anti-tyrosinase antibodies are known in the art, and novel anti-tyrosinase antibodies can be generated using well-known techniques. *Id*.

In a non-limiting method of screening for compounds that inhibit melanogenesis, melanogenic cells are grown or incubated in medium containing a test compound. The presence, concentration, or amount of tyrosinase in the medium is determined using a protein detection assay as described above. Test compounds that cause treated cells to secrete more tyrosinase than similar cells grown or incubated under similar conditions but without the test compound are candidates for compounds that inhibit melanogenesis.

Another type of assay that can be used in this screen determines the cellular localization of tyrosinase protein. In wild-type melanogenic cells, most tyrosinase is targeted to the melanosomal membrane, while some tyrosinase is secreted. Mutations or compounds that inhibit melanogenesis (e.g., mutations or compounds that inhibit P protein function) can cause tyrosinase to be secreted to the medium in greater amounts or to be mislocalized to non-melanosomal vesicles. These non-melanosomal vesicles can be separated from melanosomes using subcellular fractionation techniques. In a non-limiting example of a method of screening for compounds that inhibit melanogenesis that uses this assay, melanogenic cells are grown or incubated in medium containing a test compound and the cells are harvested. The subcellular distribution of tyrosinase is then determined in these cells and compared to the subcellular distribution of tyrosinase in similar cells grown or incubated under similar conditions but without the test compound. The assay can incorporate any technique or combination of techniques known in the art, including subcellular fractionation (for example, by sucrose or Percoll density gradient centrifugation), Western blotting of the cells' contents, and tyrosinase activity assays of each subcellular fraction. A decrease in the fraction of total tyrosinase protein found in the melanosomal fraction, or an increase in the fraction of total tyrosinase protein found in a non-melanosomal fraction, in cells treated with the test compound relative to cells not treated with the test compound indicates that the test compound inhibits melanogenesis.

Other qualitative assays can be used, such as, e.g., microscopic examination of cells treated with the test compound. For example, cell staining techniques, as known in the art, can be used. Cells are grown or incubated in medium containing tyrosine and in the presence of a test compound. The cells are stained using anti-tyrosinase antibodies, then examined microscopically. In a non-limiting example of a method of screening using this type of assay, melanogenic cells are grown or incubated in medium containing a test compound, and prepared for cell staining using techniques commonly known in the art. *See, e*.*g*., Harlow and Lane, 1988, above. Prepared cells are stained using anti-tyrosinase antibodies. The anti-tyrosinase antibodies can be conjugated to a moiety allowing for its detection. Preferably, a secondary antibody is used. The secondary antibody recognizes and binds to the anti-tyrosinase antibody. Preferably, the secondary antibody is conjugated to a moiety allowing for its detection. Alternatively, a tertiary antibody can also be used. The tertiary antibody is preferably conjugated to a moiety allowing for its detection. Examples of moieties allowing for the detection of antibodies include fluorescent molecules (for example, fluoroscein, rhodamine, Hoechst 33258, or Texas red), enzymes (for example, horseradish peroxidase, alkaline phosphatase, or beta-galactosidase), gold particles, radioactive isotope, and biotin. An assay is selected based on the labeling moiety used. For example, fluorescence microscopy can be used to detect fluorescently labeled antibodies. For cells stained with enzyme-conjugated antibodies, the cells are further treated with an appropriate substrate for conversion by the antibody-bound enzyme, followed by examination by light microscopy. Gold-particle labeled antibodies can be detected using light or electron microscopy. Isotope-labeled antibodies can be detected using radiation-sensitive film. For cells stained with biotin-conjugated antibodies, the cells are further treated with streptavidin or avidin. The streptavidin or avidin is conjugated to a moiety that allows for detection such as, for example, a fluorescent molecule, an enzyme, gold particles, or radioactive isotope. Preferably, the cells are co-stained with an antibody or antibodies specific for particular subcellular compartments *(e.g.,* endosomes, lysosomes, melanosomes, etc.). Using any one of these techniques, or any other known technique for detecting antibodies in antibody-stained cells, the subcellular distribution of tyrosinase can be determined. If the test compound causes an increased amount of tyrosinase to be found in non-melanosomal vesicles, and less tyrosinase in melanosomes, then it inhibits melanogenesis.

Another type of assay that can be used determines the presence or absence of the C-terminal portion of the tyrosinase protein. This assay depends, in part, on the discovery that melanogenic cells inhibited for melanogenesis (*e.g*., by mutations or compounds that inhibit P protein function) contain and secrete a version of tyrosinase that lacks the C-terminal portion of tyrosinase, including its transmembrane domain and its protein sorting signal. As explained above, this truncated form of tyrosinase nonetheless retains catalytic activity. In a non-limiting example of a method of screening based on this assay, melanogenic cells are grown or incubated in the presence of a test compound. An assay is selected that allows the length and/or mass of tyrosinase protein to be determined. For example, Western blots or other immunohistochemical techniques using antibodies that recognize the N-terminal or central portions of the tyrosinase protein, or other standard molecular biological techniques useful for the determination of protein length or mass, can be performed on extracts of these cells and/or on their growth or incubation medium. Antibodies appropriate for these assays can be prepared using standard immunological techniques. See, e.g., Harlow and Lane, 1988, above. If the assay reveals the presence of a shorter or lower molecular weight form of tyrosinase, relative to similar cells grown or incubated under similar conditions but without the test compound, then the test compound inhibits melanogenesis. Alternatively, Western blots or other immunohistochemical techniques using antibodies recognizing the C-terminal portion of tyrosinase *(e.g.,* the anti-PEP7 antibody prepared as described in Jimenez *et al*., 1991, J. Biol. Chem. 266:1147-1156) can be used in the assay. In these assays, a reduction in the amount of tyrosinase protein detected by the antibodies indicates that the test compound inhibits melanogenesis, because the truncated tyrosinase lacks the sequences recognized by the antibodies.

Both full length tyrosinase, and the truncated tyrosinase found in and secreted by melanogenic cells with inhibited or absent P protein, remain catalytically active when run on non-denaturing polyacrylamide gels. This observation is the basis, in part, of another assay for the truncated tyrosinase protein. Thus, melanogenic cells can be grown or incubated in medium containing a compound to be tested. Either the growth or incubation medium is collected, or cell extracts are prepared, and subjected to non-denaturing polyacrylamide gel electrophoresis. Smaller, more flexible proteins will migrate farther than larger proteins with more complicated three-dimensional structure. Filter paper or a membrane (*e.g.*, nitrocellulose) is soaked in L-DOPA and applied to the gel. Active tyrosinase in the gel converts L-DOPA into melanin, creating dark spots on the filter or membrane indicating the location, and therefore the relative size, of tyrosinase. If cells treated with the test compound produce two spots on the filter or membrane, wherein one spot indicates tyrosinase of the same size as produced by similar cells grown under similar conditions but without the test compound, and the other spot indicates tyrosinase of smaller relative size, then the test compound is a candidate for a compound that inhibits melanogenesis.

Full length tyrosinase in wild-type melanogenic cells with normal P protein function is found primarily in the insoluble fraction of cell extracts. To be released, it must be solubilized with a detergent *(e.g.,* Triton X-100™). In contrast, the smaller truncated version of tyrosinase in melanogenic cells with inhibited P protein function is found in vesicles in the soluble fraction. These observations are the basis, in part, of another assay that can be used to detect truncated tyrosinase in P protein-compromised cells. Thus, melanogenic cells are grown or incubated in medium containing a compound to be tested. The cells are harvested and can be subjected to a detergent phase separation to separate membrane-anchored proteins from soluble proteins. For example, the cells can be solubilized on ice in a buffer containing Triton X-114™. Insoluble contaminants can be spun out at 4°C. Then the supernatant, which contains solubilized proteins, is phase- separated at room temperature or elevated temperatures into a detergent phase and an aqueous phase. The ratio of tyrosinase in the detergent phase (which will contain tyrosinase proteins containing the C-terminal portion of the protein which anchors tyrosinase in the membrane) to tyrosinase in the aqueous phase (which will contain tyrosinase proteins which lack the C-terminal portion) is determined. Alternatively, cells are harvested and membranes disrupted by a freeze/thaw cycle or cycles. The disrupted cells are then separated into a soluble fraction and a membrane-bound, insoluble fraction. The ratio of soluble tyrosinase in the soluble fraction to insoluble, membrane-bound tyrosinase in the membrane fraction is determined. If cells treated with the test compound have higher levels of soluble tyrosinase than insoluble, membrane-bound tyrosinase than that from similar cells grown under similar conditions but without the test compound, then the test compound is a candidate for a compound that inhibits melanogenesis.

### 5.1.1.3 Other Methods of Screening for Inhibitors of Melanogenesis

As described above, the mislocalization and secretion of tyrosinase, and the reduction of tyrosinase activity, are not the only results of an inhibition of melanogenesis. Other melanogenic enzymes are also affected, as is the biogenesis of melanosomes.

Inhibition of melanogenesis by mutations that inhibit P protein function can cause a marked alteration in the amount of several melanogenic proteins produced in melanocytes, including the TRP-1, TRP-2, and LAMP-1 gene products (Orlow, S.J., and Brilliant, M.H., 1999, above). In the eyes of wild-type mice, the levels of TRP-1 and TRP-2 gene products are high at birth, fall sharply, increase gradually to another peak at about 2 weeks, then permanently fall to undetectable levels by about 40 days. In mice that lack P protein function, for example, the levels of these proteins are much lower at birth and are undetectable after only a few days (*id*.).

Another effect of inhibited melanogenesis caused by a mutation which inhibits P protein function is the disruption of a high molecular weight complex comprising tyrosinase, TRP-1 protein, and TRP-2 protein (Orlow, S.J. *et al*., 1994, above). For purposes of the present invention, the term "high molecular weight complex" is defined as a group of proteins bound to each other via covalent and/or non-covalent bonds that remain associated with each other during non-denaturing gel filtration, HPLC, or sucrose gradient sedimentation and have an apparent molecular weight of between about 200 kD and about 700 kD. In wild-type melanogenic cells, this "melanogenic complex." which is associated with the melanosome, contains a significant fraction of the cells' complement of tyrosinase, TRP-1 protein and TRP-2 protein. In melanogenic cells inhibited for melanogenesis by inhibition of P protein function, very little of any of these proteins is found in high molecular weight complexes.

Another assay takes advantage, in part, of these effects. In a non-limiting example of a method of screening for compounds that inhibit melanogenesis that uses this type of assay, melanogenic cells are grown or incubated in medium containing a compound to be tested. The cells are harvested, disrupted, and fractionated. This fractionation can be done, for example, using sucrose gradient sedimentation. Aliquots from the sedimentation are assayed for the presence of melanogenic proteins such as, for example, by using assays for tyrosinase, TRP-1, and/or TRP-2 activity, or by using immunohistochemical assays, such as immunoblotting. Increased amounts of any of these three proteins in low density aliquots and/or decreased amounts of any of these three proteins in high density aliquots, relative to similar cells grown or incubated under similar conditions but without the test compound, indicate that the test compound is a candidate for a compound that inhibits melanogenesis.

Another consequence of inhibited melanogenesis can be the aberrant development of melanosomes. Wild-type melanogenic cells typically contain abundant, fully developed, darkly pigmented melanosomes. Such fully developed, darkly pigmented melanosomes are less abundant or absent in melanocytes inhibited for melanogenesis due to a mutation in the P protein-encoding gene when they are grown or incubated in medium containing low concentrations of tyrosine. Rather, these cells contain an unusually large number of immature melanosomes. This phenomenon is the basis, in part, for another assay that can be used. In a non-limiting example of a method of screening for compounds that inhibit melanogenesis that uses this type of assay, melanogenic cells are grown or incubated in medium containing a test compound. The number, size, shape, and/or color of the melanosomes in the cells is assayed. Such assays are well known in the art. For example, cells can be fixed and stained and examined using light microscopy. Alternatively, cells can be fixed, stained, sectioned, and examined using electron microscopy. Alternatively, cells can be fractionated using density centrifugation. Mature melanosomes are denser than immature melanosomes, and so can be separated from them on the basis of density using well known techniques. Cells treated with a test compound that have melanosomes that are altered in number, size, shape, and/or color compared to melanosomes from similar cells grown or incubated under similar conditions but without the test compound indicates that the test compound inhibits melanogenesis.

### 5.1.1.4 Methods of Screening for Inhibitors of P Protein Function

As explained above, the P protein plays a pivotal role in melanogenesis. Melanocytes with loss of function mutations in the P protein-encoding gene are inhibited for melanogenesis. In P deficient or P inhibited cells, inhibition of melanogenesis is correlated with mislocalization of tyrosinase. Whereas in wild-type melanocytes tyrosinase is localized primarily to melanosomes, in melanocytes with loss of function mutations in the P protein-encoding gene, tyrosinase is predominantly secreted or found in non-melanosomal vesicles. Inhibition of melanogenesis and the mislocalization of tyrosinase can be mimicked by treating wild-type melanocytes with compounds that inhibit the function of P protein (*e.g.*, imipramine).

These discoveries are the basis, in part, for a number of screens for inhibitors of melanogenesis. These screens serve to identify inhibitors of melanogenesis by identifying inhibitors of P protein function. Thus, melanogenic cells are grown or incubated in medium containing a compound to be tested for its ability to inhibit P protein function. The effect, if any, of the compound can be determined using, for example, any one of the assays described above. In a non-limiting embodiment, the activity of tyrosinase in the growth or incubation medium of the cells can be measured. For example, tyrosine can be added to the medium, and its conversion to melanin monitored. Alternatively, non-tyrosine or altered tyrosine substrates of tyrosinase can be added to the medium, and their conversion into reaction products by tyrosinase can be followed by, for example, colorimetric assays (*e.g*., the DOPA oxidase assay), radiometric assays (*e.g.*, the radiometric hydroxylase or radiometric melanin synthesis assays), fluorescence assays, or by the precipitation of reaction products. These assays are described in detail in Section 5.1.1.1, above.

Alternatively, the assays of tyrosinase protein may be used. These assays can measure, for example, the amount of tyrosinase in the growth or incubation medium of the cells treated with the compound to be tested, the cellular localization of tyrosinase (*e*.*g*., by subcellular fractionation of the cells, or by staining and microscopic examination of the cells), or the length or mass of the tyrosinase molecules within the cells. These assays are described in detail in Section 5.1.1.2, above.

Other assays that can be used include those that measure other effects of the inhibition of P protein function. For example, these assays can measure the amount or activity of TRP-1 and/or TRP-2 protein in cells treated with the compound to be tested, the abundance or composition of the high molecular weight melanogenic complex, or the presence or absence of aberrant melanosomes. These assays are described in detail in Section 5.1.1.3, above.

Still another assay that can be used involves measuring the intracellular targeting, intracellular levels and/or secretion of a certain class of lysosomal hydrolases. Normally, newly synthesized lysosomal hydrolases are transported from the *trans*-Golgi network to a late endosome compartment, portions of which are thought to eventually fuse with or form lysosomes. These lysosomes, containing most of the intercellular lysosomal hydrolase activity, can be detected in a large granule fraction prepared from fractionated cells. As illustrated below by way of a non-limiting example, some, but not all, lysosomal hydrolases are not correctly targeted to the lysosome-containing large granule fraction from melan-p cells as opposed to melan-a cells. In particular, lysosomal hydrolases that are transported from the *trans*-Golgi network to the late endosome via binding to the mannose-6-phosphate/insulin-like growth factor type II receptor (the "M6P/IGF-II receptor") do not accumulate in the large granule fraction. Such incorrectly targeted lysosomal hydrolases include β-hexosaminidase, β-glucosidase, β-glucuronidase and β-galactosidase. In contrast, acid phosphatase, which is not transported to the late endosome via the M6P/IGF-II receptor, correctly accumulates in the large granule fraction in both melan-a and melan-p cells. Thus, P protein function is also necessary for the correct targeting of lysosomal enzymes that are transported to the late endosome via the M6P/IGF-II receptor. The default pathway for such enzymes is secretion into the exterior of the cell.

These results are the basis, in part, for additional methods of screening for compounds that affect P protein function. Accordingly, in lieu of, or in addition to, assays for the mislocalization of tyrosinase, one can screen for the effect of a test compound on the level and/or localization of any lysosomal hydrolase that is normally transported to the late endosome via the M6P/IGF-II receptor, including but not limited to β-hexosaminidase, β-glucosidase, β-glucuronidase and β-galactosidase. Since these lysosomal hydrolases are, like tyrosinase, proteins and more particularly enzymes, any of the methods described above to assay for the presence of tyrosinase's enzymatic activity and/or protein can be adapted to assay lysosomal hydrolases. Assays for the enzymatic activity of these enzymes are well known in the art (and, in part, illustrated below by way of non-limiting example), as are their amino acid structures and antibodies that recognize the same. For example, one can assay for the presence and/or levels of lysosomal hydrolases in whole cells or cell extracts, in the large granule fraction of a cell extract, and/or in the medium from cells treated with test compounds. Compounds that cause either a decrease in accumulation of such lysosomal enzymes in cells or, more particularly, the large granule fraction, or an increase in secretion of such lysosomal enzymes, are candidates for compounds that inhibit the function of P protein; such candidate compounds are then further analyzed using one of the other methods of the invention.

### 5.1.2 Methods of Screening for Compounds that Increase Melanogenesis, Increase P Protein Function and/or Mimic P Protein Function

As explained above, wild-type melanogenic cells typically secrete a portion of their tyrosinase into *in vitro* culture medium. Although the secreted tyrosinase is enzymatically active, it cannot contribute to melanogenesis, which occurs inside the cells' melanosomes. As described above, the level of melanogenesis within melanogenic cells is proportional to the fraction of tyrosinase that is localized to melanosomes. Compounds that decrease the amount of tyrosinase localized to melanosomes serve to inhibit melanogenesis. Conversely, compounds that reduce the amount of tyrosinase that is secreted, and thereby increase the amount of tyrosinase localized to melanosomes, are expected to increase melanogenesis. As explained above, P protein activity is required for the localization of tyrosinase to melanosomes. Thus, compounds that increase the activity of P protein in melanogenic cells, as well as compounds that mimic the activity of P protein, will increase melanogenesis by reducing the amount of tyrosinase that is secreted.

A number of screens based, in part, on these observations and predictions can be used to identify compounds that increase melanogenesis, increase the function of P protein, and/or mimic the function of P protein. For example, variations of the assays described above using melanogenic cells to identify inhibitors of melanogenesis and P protein function can be used. Melanogenic cells are grown or incubated *in vitro* in medium containing a compound to be tested for its ability to increase melanogenesis, increase P protein function or mimic P protein function. The effect, if any, of the compound can be determined using, for example, any one of the assays described above. For example, the activity of tyrosinase in the growth or incubation medium of the cells can be measured. A decrease in the activity of tyrosinase in the medium may indicate that less tyrosinase is being secreted, and that the compound might therefore increase melanogenesis or P protein function.

Alternatively, or in addition, melanogenic cells that do not contain P protein *(e.g.,* melan-p cells) can be used to screen for compounds that mimic P protein function. In one type of assay that can be used in the invention, melanogenic cells that do not contain P protein are incubated in medium containing a compound to be tested for its ability to mimic P protein function and increase melanogenesis. In contrast to normal melanogenic cells, such melanogenic cells that do not contain P protein are light colored in culture (as well as in the animal). If the melanogenic cells that do not contain P protein turn darker in the presence of the compound than in the absence of the compound, then the compound mimics P protein function in whole or in part. The color of the cells can be measured qualitatively such as, for example, by visual inspection, or quantitatively, such as, for example, by reflectance. Alternatively, melanogenic cells that do not contain P protein are treated with the compound to be tested, and the amount of tyrosinase secreted into the medium is assayed. If the amount of tyrosinase in the medium from melanogenic cells that do not contain P protein (*e.g.*, melan-p cells) decreases when the cells are treated with the test compound, then the test compound is a candidate for a compound that mimics P protein function. Tyrosinase activity in the medium can be measured, for example, by using any of the techniques described above. For example, tyrosine can be added to the medium, and its conversion to melanin monitored.

Alternatively, assays of tyrosinase protein may be used. These assays can measure, for example, the amount of tyrosinase in the growth or incubation medium of the cells treated with the compound to be tested, the cellular localization of tyrosinase (*e.g.*, by subcellular fractionation of the cells, or by staining and microscopic examination of the cells), or the length or mass of the tyrosinase molecules present within the cells. A decrease in the amount of tyrosinase protein secreted into the medium, or an increase of tyrosinase protein in melanosomes, indicates that the test compound is a candidate for a compound that increases melanogenesis or mimics or enhances P protein function. If the test compound causes similar effects in melanogenic cells that do not contain P protein (*e.g*., melan-p cells), such a result would indicate that the compound mimics P protein function. These assays are described in detail in Section 5.1.1.2, above.

Other assays that can be used include those that measure other effects of an increase in P protein function, mimic of P protein function, and/or an increase in melanogenesis. For example, these assays can measure the amount of TRP-1 and/or TRP-2 protein or activity in cells treated with the compound to be tested, the abundance or composition of the high molecular weight melanogenic complex, or the presence or absence of aberrant melanosomes as described above. An increase in the amount of TRP-1 and/or TRP-2 protein or activity, an increase in the amount of these proteins found in high molecular weight melanogenic complexes, or an increase in the number of high molecular weight complexes, indicates that the test compound is a candidate for a compound that increases melanogenesis or P protein function. If the test compound causes similar effects in melanogenic cells that do not contain P protein (*e.g*., melan-p cells), such a result would indicate that the compound mimics P protein function. These assays are described in detail in Section 5.1.1.3, above.

In a variation of these screens, the amount of secreted tyrosinase is compared to the amount of intracellular tyrosinase. Melanogenic cells are grown or incubated in medium containing a compound to be tested. Using, for example, any of the assays described above, the amount of tyrosinase in the growth or incubation medium is determined and the amount of tyrosinase within these cells is also determined. The ratio of intracellular tyrosinase to secreted tyrosinase is then calculated. If this ratio is higher for cells treated with the compound to be tested than for similar cells grown under similar conditions but without the compound, then the compound increases melanogenesis. In a non-limiting preferred embodiment, such a change in the ratio of intracellular tyrosinase to secreted tyrosinase is observed without a change (*e.g.*, reduction) in the total amount of tyrosinase produced by the cell. Similarly, if melanogenic cells that do not contain P protein (*e.g.*, melan-p cells) are grown or incubated in medium containing the compound to be tested, and the ratio of intracellular tyrosinase to secreted tyrosinase is higher for cells treated with the compound than for untreated cells, then the compound can mimic P protein function, and thereby increase melanogenesis.

### 5.1.3 Methods of Screening for Compounds That Affect P Protein Function Using Non-Melanogenic Cells

Most non-melanogenic cells do not express P protein or tyrosinase. For purposes of the present invention, the term "non-melanogenic cells" is defined as cells that do not contain melanosomes. However, non-melanogenic cells can be made to express both P protein and tyrosinase, and to synthesize melanin. For purposes of the present invention, the term "cells made to express both P protein and tyrosinase" is defined as cells that do not ordinarily express P protein and/or tyrosinase, but are caused to express both P protein and tyrosinase using any technique known in the art such as, *e.g*., molecular genetic techniques. For example, heterologous tyrosinase and/or P protein genes can be introduced into the cells by, *e.g.,* transfection, transformation, or transduction. For purposes of the present invention, the term "heterologous" is defined as describing a gene or gene product that does not naturally exist in that organism, or a gene or gene product that is not normally expressed in that cell type. Alternatively, endogenous, but normally quiescent, tyrosinase and/or P protein-encoding genes can be activated to express tyrosinase and/or P protein (*e.g.* through targeted homologous recombination of transcriptional control sequences, or any other activation method). Several methods of the present invention are based, in part, on the discovery that non-melanogenic cells expressing P protein and tyrosinase together have almost four times as much tyrosinase activity as cells expressing tyrosinase alone. Cells expressing P protein, but not tyrosinase, do not have detectable tyrosinase activity, showing that P protein' s effect on tyrosinase activity in these cells is completely dependent on the expression of tyrosinase.

The tyrosinase activity of cells made to express both tyrosinase and P protein is sensitive to the action of compounds that inhibit P protein function. When these cells are treated with, for example, imipramine, the tyrosinase activity of these cells is markedly reduced. The effect of these compounds on tyrosinase activity is totally dependent on the presence of active P protein. Cells expressing tyrosinase but not P protein have tyrosinase activities that are unaffected by the presence of the compound at the concentrations tested.

These observations are exploited in a number of methods of screening for compounds that affect (e.g., decrease or increase) P protein function. Cells that do not otherwise have detectable tyrosinase and/or P protein are made to express both of these proteins. The cells are grown or incubated in medium that contains a compound to be tested. The tyrosinase activity of extracts of these cells is measured. Tyrosinase activity can be measured using any of the assays discussed above, including the radiometric tyrosine hydroxylase assay, colorimetric DOPA oxidase assay, the DHICA converting assay, an assay for the ability to convert [¹⁴C]DOPA into TCA precipitable material, or by any other method known in the art. If the tyrosinase activity of the extracts of cells treated with the test compound is lower than the tyrosinase activity of the extracts of similar cells grown under similar conditions but without the test compound, and if the compound does not otherwise decrease tyrosinase activity in the extracts of cells expressing tyrosinase but not P protein, then the compound decreases P protein function. Conversely, if the tyrosinase activity of the extracts of cells treated with the test compound is higher than the tyrosinase activity of the extracts of similar cells grown under similar conditions but without the test compound, and if the compound does not otherwise increase tyrosinase activity in the extracts of cells expressing tyrosinase but not P protein, then the compound increases P protein function.

Another method of screening using non-melanogenic cells made to express tyrosinase and P protein exploits, in part, the discovery that these cells, if incubated long enough, turn black with melanin deposition. Cells expressing tyrosinase and P protein, or tyrosinase but not P protein, are treated with a compound to be tested. The cells are incubated for a period of time sufficient to allow cells expressing both tyrosinase and P protein, but which are not treated with the test compound, to accumulate melanin. The melanin content of treated and untreated cells can be assayed by visual inspection or spectrophotometric analysis of the cells, or by using other techniques well known in the art. If the melanin content of the cells expressing both tyrosinase and P protein and treated with the test compound is lower than the melanin content of similar cells not treated with the compound, then the compound can decrease melanogenesis. If the melanin content of cells expressing tyrosinase but not P protein is not substantially altered by the presence or absence of the compound, then the compound inhibits P protein function. Conversely, compounds that cause an increase in melanin formation in these cells, relative to similar cells grown under similar conditions but without the compound, increase melanogenesis. If the compound also fails to increase melanin formation in non-melanogenic cells expressing a tyrosinase-encoding gene but not a P protein-encoding gene, then the compound increases P protein function.

Alternatively, broken cell extract systems can be devised to study intracellular trafficking of tyrosinase. In a non-limiting example, donor Golgi membranes and cytosol from wild-type melanocytes can be combined with melanosomes prepared from cells of a mouse with a mutation in the tyrosinase gene that inactivates the enzyme. One could then observe the transfer of tyrosinase from the wild-type donor Golgi membranes to the tyrosinase-deficient melanosomes. Addition of a compound that inhibits P protein function would inhibit such transfer.

For those methods using heterologous genes, the heterologous tyrosinase and P protein-encoding genes can be derived from any suitable source. Preferably, they are derived from an animal source. More preferably, they are derived from a mammalian source such as the mouse cells used below in illustrating embodiments. Even more preferably, they are derived from a primate source such as humans. Tyrosinase-encoding genes are well known in the art (*see,* for example, expression of the human gene cDNA in Bouchard *et al*., 1989, J. Exp. Med. 169 (6), 2029-2042, and the MEDLINE database at accession nos., for example, NM_000372, M27160, and U01873), as are P protein-encoding genes (see for example, Rinchik *et al*., 1993, Nature 361 (6407), 72-76, and the MEDLINE database at accession nos., for example, NM_000275 and U19152 for the human gene).

Expression cassettes are typically used to express heterologous genes in the chosen cell. Each expression cassette contains regulatory sequences designed to express, for example, the tyrosinase-encoding gene and/or the P protein-encoding gene. For expression in prokaryotic cells, preferably each coding sequence found in the expression cassette is operatively linked to at least one regulatory sequence, *i*.*e*., a promoter sequence. By "operatively linked" is meant that the regulatory sequence functions to regulate the coding sequence *(e.g.,* controls the timing or amount of expression of the coding sequence, determines initiation or termination of transcription or translation, or affects message stability). For expression in eukaryotic cells, preferably each coding sequence found in the expression cassette is "operatively linked" to at least two regulatory sequences, *i*.*e*., a promoter and a polyA sequence. Each expression cassette is operatively linked to the polynucleotide sequence of a vector. Each vector preferably contains polynucleotide sequences that allow for its selection, replication, and maintenance in transfected cells, either as an autonomous extrachromosomal element, or as an integrated component of one or more chromosomes in the transfected cells. Vectors containing expression cassettes that can be adapted to express almost any coding sequence are well known in the art and commercially available. Non-limiting examples of such vectors are illustrated below using the pcDNA vectors available from Invitrogen (San Diego, CA).

Any promoter that facilitates a sufficiently high rate of expression can be used in the expression cassette. The promoter can be constitutive or inducible. See, e.g., Resendez et al., 1988, Mol. Cell Biol. 8:4579-4584; and Chang et al., 1987, Proc. Natl. Acad. Sci. USA 84:680-684, which describe inducible promoters. The choice of the promoter depends on what cell type is used in the screen and the desired level of expression of the heterologous genes encoding tyrosinase and/or P protein. *See, e.g.*, Gossen *et al*., 1995, Science 268:1766-1769; Gossen and Bujard, 1992, Proc. Natl. Acad. Sci. USA 89:5547-5551 and U.S. Patent Nos. 5,851,984; 5,849,997; 5,827,687; 5,811,260; 5,789,215; 5,665,578; 5,512,483; 5,302,517; 4,959,313; and 4,935,352, which describe useful promoter sequences.

Further non-limiting examples of promoter sequences and elements include the SV40 early promoter region (Bernoist and Chambon, 1981, Nature 290:304-310), the promoter contained in the 3' long terminal repeat of Rous sarcoma virus (Yamamoto, *et al*., 1980, Cell 22:787-797), the herpes thymidine kinase promoter (Wagner *et al*., 1981, Proc. Natl. Acad. Sci. U.S.A. 78:1441-1445), the regulatory sequences of the metallothionein gene (Brinster *et al*., 1982, Nature 296:39-42); prokaryotic expression vectors such as the β-lactamase promoter (Villa-Kamaroff, *et al*., 1978, Proc. Natl. Acad. Sci. U.S.A. 75:3727-3731), and the tac promoter (DeBoer, *et al*., 1983, Proc. Natl. Acad. Sci. U.S.A. 80:21-25); see also "Useful proteins from recombinant bacteria" in Scientific American, 1980, 242:74-94; plant expression vectors comprising the nopaline synthetase promoter region (Herrera-Estrella *et al*., Nature 303:209-213) or the cauliflower mosaic virus 35S RNA promoter (Gardner, *et al*., 1981, Nucl. Acids Res. 9:2871), and the promoter of the photosynthetic enzyme ribulose biphosphate carboxylase (Herrera-Estrella *et al*., 1984, Nature 310:115-120); promoter elements from yeast or other fungi such as the Gal 4 promoter, the ADC (alcohol dehydrogenase) promoter, PGK (phosphoglycerol kinase) promoter, alkaline phosphatase promoter, and the following animal transcriptional control regions, which exhibit tissue specificity and have been utilized in transgenic animals: the elastase I gene control region, which is active in pancreatic acinar cells (Swift *et al*., 1984, Cell 38:639-646; Ornitz *et al*., 1986, Cold Spring Harbor Symp. Quant. Biol. 50:399-409; MacDonald, 1987, Hepatology 7:425-515); the insulin gene control region, which is active in pancreatic beta cells (Hanahan, 1985, Nature 315:115-122); the immunoglobulin gene control region, which is active in lymphoid cells (Grosschedl *et al*., 1984, Cell 38:647-658; Adames *et al*., 1985, Nature 318:533-538; Alexander *et al*., 1987, Mol. Cell. Biol. 7:1436-1444); the mouse mammary tumor virus control region, which is active in testicular, breast, lymphoid and mast cells (Leder *et al*., 1986, Cell 45:485-495); the albumin gene control region, which is active in liver (Pinkert *et al.,* 1987, Genes and Devel. 1:268-276); the alpha-fetoprotein gene control region, which is active in liver (Krumlauf *et al*., 1985, Mol. Cell. Biol. 5:1639-1648; Hammer *et al.,* 1987, Science 235:53-58); the alpha 1-antitrypsin gene control region, which is active in the liver (Kelsey *et al*., 1987, Genes and Devel. 1:161-171); the beta-globin gene control region, which is active in myeloid cells (Mogram *et al*., 1985, Nature 315:338-340; Kollias *et al*., 1986, Cell 46:89-94); the myelin basic protein gene control region, which is active in oligodendrocyte cells in the brain (Readhead *et al*., 1987, Cell 48:703-712); the myosin light chain-2 gene control region, which is active in skeletal muscle (Sani, 1985, Nature 314:283-286); and the gonadotropic releasing hormone gene control region, which is active in the hypothalamus (Mason *et al*., 1986, Science 234:1372-1378).

Another regulatory element that can be used in the expression cassette for eukaryotic cell expression is a polyA sequence (or polyA signal), which should be capable of efficiently inducing polyadenylation of a transcript specific for the coding sequence to which the polyA sequence is operatively linked. *See, e.g.,* U.S. Patent Nos. 5,861,290; 5,851,984; 5,840,525 and 5,627,033, which discuss polyA sequences.

In another non-limiting embodiment, the expression cassette used according to the present invention may further comprise an enhancer element, a 5' or 3' untranslated sequence (or region), one or more introns, a sequence that regulates RNA stability, or a combination of more than one of these elements. Any sequence that falls into any of these categories can be used in the vector of the present invention. See U.S. Patent Nos. 5,861,290; 5,851,984; 5,840,525; 5,681,744 and 5,627,033, which discuss these regulatory elements. The term "5' untranslated sequence" refers to the sequence of an mRNA molecule between the transcription initiation site and the translation initiation site. The term "3' untranslated sequence" refers to the sequence of an mRNA molecule between the translation termination site and the polyA tail.

The heterologous genes used in these assays are typically introduced into the chosen cells by transfection, transduction, transformation, or any other suitable technique known in the art. For example, electroporation, calcium phosphate coprecipitation, microinjection, lipofection, etc., can be used. *See, e.g.,* U.S. Patent No. 5,814,618 and 5,789,215, which describe transfection methods. The cells that take up the heterologous gene or genes, either through integration into their genome or by maintenance as part of an extrachromosomal element, are then preferably selected by standard techniques. Thus, a selectable marker can be included in the vector which allows a cell that has the marker, and thus cells that contain the vector and the heterologous gene or genes, to be isolated from cells that do not have the marker. Whether a selectable marker is necessary to prepare the cells used in these assays depends on the particular method by which the vector is introduced into the cells. For example, if the vector is introduced into the cells via microinjection, a.selectable marker may be less useful than if electroporation is used because the transformation frequency tends to be higher for microinjection. For example, the marker can enable a cell to grow under selective conditions, *i*.*e*. conditions under which the cell could not grow if it did not have the marker *(e.g.,* the neomycin resistance gene and the hypoxanthine phosphoribosyltransferase gene). A marker can also provide another means by which to identify the cell which took up the heterologous polynucleotide molecule or vector (*e.g.*, by preferential staining). *See, e.g.*, U.S. Patent Nos. 5,851,984 and 5,789,215, which describe selectable markers.

The cells used in these assays can typically be derived from any source. The cells used in these assays can be cells derived from a mammalian animal, for example: a sheep, cow, pig, or other farm animal; a cat, dog, or other domesticated animal; a mouse, rat, or other rodent; a monkey, ape, or other primate; and most preferably a human. Alternatively, the cells used in these assays can be derived from non-mammalian animals such as, for example, a bird, fish, reptile, amphibian, or insect. Cells derived from animals for use in these assays can be of any type such as, for example, fibroblasts, glial cells, keratinocytes, hepatocytes, ependymal cells, bone marrow cells, hippocampal cells, stem cells, embryonic stem cells, hematopoietic stem cells, olfactory mucosa cells, adrenal cells, leukocytes, lymphocytes, chromaffin cells, neurons, cells of the immune system, macrophages, Schwann cells, oligodendrocytes, astrocytes, germline cells, somatic cells, epithelial cells, endothelial cells, adrenal medulla cells, osteoblasts, osteoclasts, myoblasts, pancreatic cells *(e.g.,* of the islets of Langerhans), or a mixture of more than one of the above cell types, etc. Alternatively, the cells used in these assays can be derived from a plant source such as, for example, a dicotyledon such as, *e.g.*, tobacco, or a monocotyledon, such as, *e.g.,* corn. Alternatively, the cells used in these assays can be derived from a unicellular eukaryotic organism such as, for example, a protozoan or a yeast or other unicellular fungus. Methods of growing these cells are specific to each cell type and within the skill of the art.

In a preferred embodiment, established cell lines from any of these sources can be used for these assays. Examples of suitable cell lines include, but are not limited to, Chinese Hamster Ovary (CHO) cells, HeLa cells, NRK cells, A293 cells, and COS cells. The cells should have the ability to proliferate when grown in *in vitro* culture. Following introduction of the heterologous gene or genes into the cells, and selection for cells that have taken up the heterologous gene or genes, such cells, in a preferred embodiment, should be useful to establish a cell line that can be grown, stored, re-grown, etc., for extended periods of time in *in vitro* culture. *See, e.g.,* U.S. Patent No. 5,814,618, which describes cells useful for the assays of the present invention.

### 5.1.4 High-Throughput Methods of Screening for Compounds that Affect or Mimic P Protein Function

The methods of screening for compounds that affect or mimic P protein function described above can be used to test individual compounds or small numbers or large numbers of compounds contemporaneously. High-throughput methods of screening, as known in the art, are preferable.

For purposes of the present invention, the term "high-throughput method of screening" is defined as a method of screening that allows for large numbers of compounds to be tested concurrently. Each or all of the steps in screening compounds that affect or mimic P protein function are amenable to high throughput methods of screening for candidate compounds. Preferably, the high-throughput methods of screening are partially or fully automated, reducing the amount of attention required to test each compound. For example, an increase in the amount of tyrosinase secreted into the medium, or total levels of tyrosinase activity, can be detected easily in the formats (such as, *e.g*., 96 well plates) typically used in high-throughput methods of screening. High-throughput methods of screening are well known in the art and can be performed in any of a number of formats. Laboratory automation, including robotics technology, can significantly decrease the time necessary to screen large numbers of compounds, and is commercially available from, for example, Tecan (Research Triangle Park, NC), Scitec Laboratory Automation SA (Lausanne, Switzerland), Rosys (New Castle, DE), Rixan Associates Inc. (Dayton, OH), CRS Robotics (Burlington, Ontario Canada), Fanuk Robotics, and Beckman-Coulter Sagian (Indianapolis, IN), to name just a few companies. Upon identifying candidate compounds, secondary methods of screening can be performed to determine the cellular and/or *in vivo* effects of the candidate compounds on P protein function.

### 5.1.5 Secondary Methods of Screening and Additional Methods of Screening for Compounds That Affect or Mimic P Protein Function

Each of the above methods of screening can be used by itself to identify compounds that are likely to affect or mimic P protein function. Alternatively, a plurality of methods of screening can be used serially to confirm, or to determine more accurately, the P protein affecting properties of one or more compounds. For example, any of the above methods of screening can be used as a primary method of screening, followed by a secondary method of screening. For purposes of the present invention, the term "primary method of screening" is defined as the first method of screening used to test the ability of a compound to affect or mimic P protein function. For purposes of the present invention, the term "secondary method of screening" is defined as any method of screening that is not the primary method of screening. The use of secondary methods of screening is particularly important when the primary method of screening is based on the identification of compounds that lower the activity of tyrosinase or the amount of melanin produced, or that lower the amount of tyrosinase secreted. Direct inhibitors of tyrosinase will also cause a reduction in the activity of tyrosinase and the amount of melanin produced, or can cause a reduction in tyrosinase activity, but would not necessarily affect P protein function.

Any of the methods of screening described above can also be used as a secondary method of screening. For example, one can identify candidate compounds using as a primary screen the assay for an effect on tyrosinase activity in cells made to express tyrosinase and P protein, yet which don't affect tyrosinase activity in cells made to express tyrosinase alone. Promising compounds from this primary screen can then be tested in a secondary screen in an assay for their effect on cellular localization of tyrosinase and/or lysosomal enzymes in melanogenic cells. Of the methods of screening described above, the ones which rely upon identification of the mislocalization of tyrosinase protein or activity or size are preferred as secondary methods of screening.

In one embodiment, a secondary screen is employed to distinguish the effects of test molecules that effect the melanogenic pathway in general and P-protein in particular, and those that inhibit protein synthesis, trafficking and proteolysis. For example, in an assay for activators of P-protein function, a secondary screen can simply entail visually examining the test melanocytes to ensure a darker color and therefore an increase in P-protein activity, rather than a general inhibition of protein synthesis, trafficking or proteolysis by the test molecule and resulting decrease in tyrosinase secretion. Alternatively, the cells can be histologically examined, preferably by electron microscopy, optionally together with DOPA staining (as described in Section 9, *infra*), to determine their melanosome content. A true activator of P-protein activity will promote the maturation of melanosomes from stages I-III to stages III-IV, whereas an inhibitor of protein synthesis, trafficking and proteolysis is unlikely to promote melanosome maturation.

Other methods of screening can be used. For example, compounds can first be screened for binding affinity to purified P protein. Alternatively, a compound identified by a primary method of screening as affecting P protein function can be tested for direct binding to purified P protein *in vitro*, or by copurification with P protein from P protein-expressing cells treated with the compound. Each of these methods of screening can determine whether the compound binds directly to P protein. A compound that can bind directly to P protein and which also affects tyrosinase activity or localization or some other aspect of melanogenesis is likely to directly affect P protein function. Alternatively, a compound identified by a primary method of screening as affecting P protein function can be tested for the ability to affect tyrosinase directly. For example, the test compound can be added to a system that contains tyrosinase but not P protein. Such a system can be, for example, an *in vitro* system containing purified or partially purified tyrosinase protein free or essentially free of P protein. Alternatively, it can be a cell that expresses tyrosinase but not P protein. If the effect of the test compound on tyrosinase is P protein independent, then the test compound does not affect P protein function. If the effect of the test compound on tyrosinase is also observed in the absence of cellular trafficking *(e.g.,* on purified tyrosinase protein, and not in cells), then the test compound does not mimic P protein function.

While preferred primary methods of screening, especially those that are high-throughput methods of screening, are those with the lowest costs (that is, can be performed as quickly, with as little human supervision, and using as few materials as possible), secondary methods of screening can be more time, labor, and material-intensive. This is because the secondary methods of screening are performed only on test compounds that are identified by the primary method of screening as affecting or mimicking P protein function. These compounds are expected to be a small fraction of the total number of compounds tested in any large scale, high-throughput screening effort. Examples of methods of screening that are better suited for secondary screens than for primary screens include administration of a test compound to an animal (*e.g.*, topically, subcutaneously, or orally) or to animal skin equivalents grown in culture, where lightening of the skin or skin equivalent indicates that the compound inhibits P protein function. Or, for compounds that mimic P protein function, the secondary screen can include administration of the test compound to a melan-p animal or animal skin equivalent, where darkening of the skin or skin equivalent indicates that the compound mimics P protein function.

Primary and secondary methods of screening can be used in another way to identify compounds that affect or mimic P protein function. Once a compound that affects or mimics P protein function is identified by using, for example, a primary method of screening, chemical analogs of the compound can be selected or created. For purposes of the present invention, the term "chemical analog" is defined as a compound that is chemically related to another chemical compound. The relationship is preferably structural as known in the art such as where, for example, the two compounds differ only in the location of a substituent, such as, *e.g.*, a hydroxyl or alkyl group, or are chemical homologs of each other. Alternatively, the relationship might be functional such as where, for example, both compounds affect the same mechanism, such as, *e.g*., where both compounds are kinase inhibitors. Methods for designing or selecting chemical analogs are described below in Section 5.2. These chemical analogs can then be tested for the ability to affect or mimic P protein function using, for example, any method described above. The secondary method of screening can be the same as the primary method of screening, or it can be a different method of screening. Chemical analogs are sought which have a stronger effect on P protein function than the original test compound. This procedure can be repeated serially to identify or create compounds of increasing efficacy.

### 5.2 Compounds for Inhibiting, Increasing or Mimicking P Protein Function

Compounds that can be screened in accordance with the present invention include but are not limited to small organic molecules that are able to gain entry into a cell and affect P protein activity. A number of compound libraries are commercially available from companies such as Pharmacopeia (Princeton, NJ), Arqule (Medford, MA), Enzymed (lowa City, IA), Sigma-Aldrich (St. Louis, MO), Maybridge (Trevillett, United Kingdom), Trega (San Diego, CA) and PanLabs (Bothell, WA), to name just a few sources. One also can screen libraries of known compounds, including natural products or synthetic chemicals, and biologically active materials, including proteins, for compounds that affect or mimic P protein function.

One class of preferred compounds for use in the methods of the present invention comprises chemical analogs of imipramine. As described above, imipramine inhibits P protein function. Imipramine is a tricyclic tertiary amine used in the treatment of depression. See Gilman, A.G. *et al*., eds, 1990, *Goodman and Gilman's The Pharmacological Basis of Therapeutics,* Eighth Edition, 405-14, Pergamon Press, New York. Other tricyclic tertiary amines used in the treatment of depression such as, for example, amitriptyline, trimipramine, or doxepin *(see id.)* can be test compounds in screens for compounds that affect P protein function. Secondary amines used in the treatment of depression such as, for example, desipramine, nortriptyline, protriptlyine, amoxapine, or maprotiline *(see id.)* also are preferred compounds for the screens of the present invention. These chemical analogs of imipramine all share structural and functional characteristics with imipramine. Other chemical analogs of imipramine that are preferred compounds for use in the methods of the present invention include chemicals with functional and/or structural similarities to imipramine. For example, the atypical antidepressants such as, for example, trazodone and fluoxetine, lack structural similarity with imipramine *(see id.),* but share the functional property with imipramine of being useful antidepressants, and so are preferred compounds for the screens of the present invention. Tricyclic compounds, tertiary amines, and secondary amines without antidepressant effects also are preferred compounds of the present invention.

Once a compound that affects or mimics P protein function is identified, molecular modeling techniques can be used to design chemical analogs of the compound that are more effective. For example, chemical analogs of imipramine, or any of the other preferred compounds listed above, can be created using these or other modeling techniques. Examples of molecular modeling systems are the CHARM (Polygen Corporation, Waltham, MA) and QUANTA (Molecular Simulations Inc., San Diego, CA) programs. CHARM performs the energy minimization and molecular dynamics functions. QUANTA performs the construction, graphic modeling and analysis of molecular structure. QUANTA allows interactive construction, modification, visualization, and analysis of the behavior of molecules with each other.

For example, once a compound that affects or mimics P protein function is identified, the compound can be used to generate a hypothesis. Such a hypothesis can be generated from any one of the preferred compounds of the present invention using, *e.g.,* the program Catalyst (Molecular Simulations Inc., San Diego, CA). Furthermore, Catalyst can use the hypothesis to search proprietary databases such as, for example, the Cambridge small molecule database (Cambridge, England), as well as other databases or compound libraries, *e.g.*, those cited above, to identify additional examples of the compounds of the present invention.

Compounds of the present invention can further be used to design more effective analogs using modeling packages such as Ludi, Insight II, C²-Minimizer and Affinity (Molecular Simulations Inc., San Diego, CA). A particularly preferred modeling package is MacroModel (Columbia University, NY,NY).

The compounds of the present invention can further be used as the basis for developing a rational combinatorial library. Such a library can also be screened to identify more effective compounds. While the nature of the combinatorial library is dependent on various factors such as the particular compound chosen from the preferred compounds of the present invention to form the basis of the library, as well as the desire to synthesize the library using a resin, it will be recognized that the compounds of the present invention provide requisite data suitable for combinatorial design programs such as C²-QSAR (Molecular Simulations Inc., San Diego, CA).

Another class of compounds that can be used to inhibit the function of P protein are P protein-encoding gene antisense nucleic acids. A P protein-encoding gene antisense nucleic acid as used herein refers to an oligonucleotide or polynucleotide molecule having a nucleic acid sequence capable of hybridizing to a portion of a P protein-encoding RNA (preferably mRNA) by virtue of some degree of sequence complementarity. The antisense nucleic acid should be complementary to either a coding and/or noncoding region of a P protein mRNA such that it inhibits P protein function by reducing the amount of P protein synthesized.

The antisense nucleic acids of the present invention can be oligonucleotides that are double-stranded or single-stranded, RNA or DNA, or a modification or analog thereof, which can be directly administered to a cell, or to the skin of an animal, or which can be produced intracellularly by transcription of heterologous, introduced sequences.

In one embodiment, the present invention is directed to methods for inhibiting the expression of a P protein-encoding nucleic acid sequence in a prokaryotic or eukaryotic cell comprising providing the cell with an effective amount of a composition comprising a P protein-encoding gene antisense nucleic acid of the present invention.

The P protein-encoding gene antisense nucleic acids of the present invention are at least about six nucleotides in length and are more preferably oligonucleotides ranging from about 6 to about 50 oligonucleotides. In specific aspects, the oligonucleotide is at least about 10 nucleotides, at least about 1.5 nucleotides, at least about 100 nucleotides, or at least about 200 nucleotides in length. The oligonucleotides can be DNA or RNA, or chimeric mixtures or derivatives, and modified versions thereof, which can either be single-stranded or double-stranded. The oligonucleotide can be modified at the base moiety, sugar moiety, or phosphate backbone level. The oligonucleotide may include other appending groups such as peptides, or agents facilitating transport across the cell membrane (see, *e.g.,* Letsinger et al., 1989, Proc. Natl. Acad. Sci. U.S.A. 86:6553-6556; Lemaitre et al., 1987, Proc. Natl. Acad. Sci. 84:648-652; PCT Publication No. WO 88/09810, published December 15, 1988), hybridization-triggered cleavage agents (see, *e.g.,* Krol et al., 1988, BioTechniques 6:958-976), or intercalating agents (see, *e.g.*, Zon, 1988, Pharm. Res. 5:539-549).

In a preferred aspect of the present invention, a P protein-encoding gene antisense oligonucleotide is a single-stranded DNA molecule. The oligonucleotide may be modified at any position on its structure with substituents generally known in the art.

The P protein-encoding gene antisense oligonucleotide may comprise at least one modified base moiety which is selected from a group including but not limited to 5-fluorouracil, 5-bromouracil, 5-chlorouracil, 5-iodouracil, hypoxanthine, xantine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5N-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2-carboxypropyl) uracil, (acp3)w, and 2,6-diaminopurine.

In another embodiment, the oligonucleotide comprises at least one modified sugar moiety selected from a group including but not limited to arabinose, 2-fluoroarabinose, xylulose, and hexose.

In yet another embodiment, the oligonucleotide comprises at least one modified phosphate backbone component selected from the group consisting of a phosphorothioate, a phosphorodithioate, a phosphoramidothioate, a phosphoramidate, a phosphordiamidate, a methylphosphonate, an alkyl phosphotriester, and a formacetal or analog thereof.

In yet another embodiment, the oligonucleotide is an alpha-anomeric oligonucleotide. An alpha-anomeric oligonucleotide forms specific double-stranded hybrids with complementary RNA in which, contrary to the usual beta-units, the strands run parallel to each other (Gautier et al., 1987, Nucl. Acids Res. 15:6625-6641).

The oligonucleotide may be conjugated to another molecule such as; *e.g.,* a peptide, hybridization triggered cross-linking agent, transport agent, hybridization-triggered cleavage agent, etc.

Oligonucleotides of the present invention may be synthesized by standard methods known in the art including, *e.g.* by use of an automated DNA synthesizer (such as are commercially available from Biosearch, Applied Biosystems, etc.). As examples, phosphorothioate oligonucleotides may be synthesized by the method of Stein et al., 1988, Nucl. Acids Res. 16:3209, and methylphosphonate oligonucleotides can be prepared by use of controlled pore glass polymer supports using the method of Sarin et al., 1988, Proc. Natl. Acad. Sci. U.S.A. 85:7448-7451, etc.

In a specific embodiment, the P protein antisense oligonucleotide comprises catalytic RNA, or a ribozyme (see, *e.g.,* PCT International Publication WO 90/11364, published October 4, 1990; Sarver et al., 1990, Science 247:1222-1225). In another embodiment, the oligonucleotide is a 2'-O-methylribonucleotide (Inoue et al., 1987, Nucl. Acids Res. 15:6131-6148), or a chimeric RNA-DNA analogue (Inoue et al., 1987, FEBS Lett. 215:327-330).

In an alternative embodiment, the P protein-encoding gene antisense nucleic acid of the invention is produced intracellularly by transcription from an heterologous sequence. For example, a vector can be introduced *in vivo* such that it is taken up by a cell, within which cell the vector or a portion thereof is transcribed, producing an antisense nucleic acid (RNA) of the invention. Such a vector would contain a sequence encoding the P protein-encoding gene antisense nucleic acid. Such a vector can remain episomal or become chromosomally integrated, as long as it can be transcribed to produce the desired antisense RNA. Such vectors can be constructed by standard recombinant DNA technology methods known in the art. Vectors can be plasmids, viral vectors, or others known in the art as useful for replication and expression in mammalian cells. Expression of the sequence encoding the P protein-encoding gene antisense RNA can be regulated by any promoter known in the art to act in such cells. Such promoters can be inducible or constitutive, and can include but are not limited to those listed above.

The antisense nucleic acids of the invention comprise a sequence complementary to at least a portion of an RNA transcript of a P protein-encoding gene, preferably a human P protein-encoding gene. However, absolute complementarity, although preferred, is not required, as long as the antisense nucleic acid has sufficient complementarity to be able to hybridize with the RNA, forming a stable duplex. In the case of double-stranded P protein-encoding gene antisense nucleic acids, a single strand of the duplex DNA may thus be tested, or triplex formation may be assayed. The ability to hybridize will depend on both the degree of complementarity and the length of the antisense nucleic acid. Generally, the longer the hybridizing nucleic acid, the more base mismatches with a P protein-encoding gene RNA it may contain and still form a stable duplex (or triplex, as the case may be). One skilled in the art can determine the mismatch tolerance by use of standard procedures to, *e.g.,* determine the melting point of the hybridized complex.

### 5.3 Methods of Inhibiting, Increasing or Mimicking P Protein Function

Compounds that affect or mimic the function of P protein can be used to treat animals or, preferably, humans that have diseases, conditions, or disorders caused by the production or overproduction of melanin. Such diseases, conditions, or disorders include those that can be characterized by discolorations of the skin or hair such as, for example, hyperpigmentation caused by inflammation or from diseases such as melasma, or brown spots such as "café au lait" macules. Alternatively, a subject may wish to lighten the color of his or her hair or skin. Compounds that increase the function of P protein or that mimic the function of P protein can be used to treat animals or, preferably, humans that have diseases, conditions, or disorders caused by the underproduction of melanin such as, for example, post-inflammatory hypopigmentation, pityriasis alba, and certain forms of albinism such as, for example, OCA II albinism. Additionally, such compounds can be used to darken the color of one's hair or skin. For the purposes of this application, the terms "treatment", "therapeutic use", and "medicinal use" shall refer to any and all uses of the compositions of the invention which remedy a disease state or one or more symptoms, or otherwise prevent, hinder, retard, or reverse the progression of disease or one or more other undesirable symptoms in any way whatsoever.

### 5.3.1 Pharmaceutical Applications

For pharmaceutical uses, it is preferred that the compound that affects or mimics P protein function is part of a pharmaceutical composition. Pharmaceutical compositions, comprising an effective amount of a compound that affects P protein function in a pharmaceutically acceptable carrier, can be administered to a patient, person, or animal having a disease, disorder, or condition which is of a type that produces, or overproduces, melanin.

The amount of compound that affects or mimics P protein function which will be effective in the treatment of a particular disease, disorder, or condition will depend on the nature of the disease, disorder, or condition, and can be determined by standard clinical techniques. Where possible, it is desirable to determine *in vitro* the cytotoxicity of the compound to the tissue type to be treated, and then in a useful animal model system prior to testing and use in humans.

The compounds that affect or mimic P protein function can be administered for the reduction or increase of melanin synthesis by any means that results in contact of the active agent with its site of action in the body of a mammal. The compounds can be administered by any conventional means available for use in conjunction with pharmaceuticals, either as individual therapeutic agents or in a combination of therapeutic agents. Each can be administered alone, but is preferably administered with a pharmaceutical carrier selected on the basis of the chosen route of administration and standard pharmaceutical practice. The pharmaceutical compositions of the invention can be adapted for oral, parenteral, topical or rectal administration, and can be in unit dosage form, in a manner well known to those skilled in the pharmaceutical art. Parenteral administration includes but is not limited to, injection subcutaneously, intravenously, intraperitoneally or intramuscularly. However, topical application is preferred.

### 5.3.2 Cosmetic Applications

In addition to pharmaceutical uses, the methods of the current invention are useful for cosmetic purposes. Cosmetic applications for methods of the present invention include the topical application of compositions containing one or more compounds that affect or mimic P protein function to enhance or otherwise alter the visual appearance of skin or hair. Occurrences in the skin or hair of noticeable but undesired pigmentation as a result of melanin production, overproduction or underproduction can be treated using the methods of the present invention.

### 5.3.3 Endpoints and Dosages

An effective dosage and treatment protocol can be determined by conventional means, starting with a low dose in laboratory animals and then increasing the dosage while monitoring the effects, and systematically varying the dosage regimen as well. Animal studies, preferably mammalian studies, are commonly used to determine the maximal tolerable dose, or MTD, of a bioactive agent per kilogram weight. Those skilled in the art can extrapolate doses for efficacy and avoidance of toxicity to other species, including humans.

Before human studies of efficacy are undertaken, Phase I clinical studies in normal subjects can help establish safe doses. Numerous factors can be taken into consideration by a clinician when determining an optimal dosage for a given subject. Primary among these is the toxicity and half-life of the chosen compound that affects or mimics P protein function. Additional factors include the size of the patient, the age of the patient, the general condition of the patient, the particular disease, condition, or disorder being treated, the severity of the disease, condition, or disorder being treated, the presence of other drugs in the patient, the effect desired, and the like. The trial dosages would be chosen after consideration of the results of animal studies and the clinical literature.

One of ordinary skill in the art will appreciate that the endpoint chosen in a particular case will vary according to the disease, condition, or disorder being treated, the outcome desired by the patient, subject, or treating physician, and other factors. Where the composition is being used to lighten or darken skin color such as, for example, to reverse hyperpigmentation caused by, for example, inflammation or diseases such as melasma, or to lighten or darken hair color, any one of a number of endpoints can be chosen. For example, endpoints can be defined subjectively such as, for example, when the subject is simply "satisfied" with the results of the treatment. For pharmacological compositions, the endpoint can be determined by the patient's, or the treating physician's, satisfaction with the results of the treatment. Alternatively, endpoints can be defined objectively. For example, the patient's or subject's skin or hair in the treated area can be compared to a color chart. Treatment is terminated when the color of the skin or hair in the treated area is similar in appearance to a color on the chart. Alternatively, the reflectance of the treated skin or hair can be measured, and treatment can be terminated when the treated skin or hair attains a specified reflectance. Alternatively, the melanin content of the treated hair or skin can be measured. Treatment can be terminated when the melanin content of the treated hair or skin reaches a specified value. Melanin content can be determined in any way known to the art, including by histological methods, with or without enhancement by stains for melanin.

### 5.3.4 Methods of Administration

The compound that affects or mimics P protein function (*i*.*e*., the active ingredient) can be administered orally in solid or semi-solid dosage forms, such as hard or soft-gelatin capsules, tablets, or powders, or in liquid dosage forms, such as elixirs, syrups, or suspensions. It can also be administered parenterally, in sterile liquid dosage forms. Since topical application is preferred, other dosage forms are potentially possible such as patches, ointments, creams, gels, lotions, solutions, suppositories or transdermal administration.

Because *in vivo* use is contemplated, the composition is preferably of high purity and substantially free of potentially harmful contaminants, *e.g.,* at least National Food (NF) grade, generally at least analytical grade, and preferably at least pharmaceutical grade. To the extent that a given compound must be synthesized prior to use, such synthesis or subsequent purification shall preferably result in a product that is substantially free of any potentially contaminating toxic agents that may have been used during the synthesis or purification procedures.

Gelatin capsules or liquid-filled soft gelatin capsules can contain the active ingredient and powdered or liquid carriers, such as lactose, lecithin starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar-coated or film-coated to mask any unpleasant taste and to protect the tablet from the atmosphere, or enteric-coated for selective, targeted disintegration in the gastrointestinal tract. Liquid dosage forms for oral administration can contain coloring and/or flavoring to increase patient acceptance.

In general, sterile water, oil, saline, aqueous dextrose (glucose), polysorbate and related sugar solutions and glycols such as propylene glycol or polyethylene glycols, are suitable carriers for parenteral solutions. Solutions or emulsions for parenteral administration preferably contain about 5-15% polysorbate 80 or lecithin, suitable stabilizing agents, and if necessary, buffer substances. Antioxidizing agents, such as but not limited to sodium bisulfite, sodium sulfite, or ascorbic acid, either alone or combined, are suitable stabilizing agents. Also useful are citric acid and its salts, and sodium EDTA. In addition, parenteral solutions can contain preservatives, including but not limited to benzalkonium chloride, methyl- or propyl-paraben, and chlorobutanol.

Suitable pharmaceutical carriers are further described in *Remington's Pharmaceutical Sciences,* 17th ed., Mack Publishing Company, Easton, PA (1990) a standard reference text in this field, which is incorporated herein by reference in its entirety.

Useful pharmaceutical dosage forms for administration of compounds that affect or mimic P protein function are described below.

For topical administration, compounds that affect or mimic P protein function can be formulated as a solution, gel, lotion, ointment, cream, suspension, paste, liniment, powder, tincture, aerosol, transdermal drug delivery system, or the like in a pharmaceutically or cosmetically acceptable form by methods well known in the art. The composition can be any of a variety of forms common in the pharmaceutical or cosmetic arts for topical application to animals or humans, including solutions, lotions, sprays, creams, ointments, salves, gels, etc. Preferred agents are those that are viscous enough to remain on the treated area, those that do not readily evaporate, and/or those that are easily removed by rinsing with water, optionally with the aid of soaps, cleansers and/or shampoos. Actual methods for preparing topical formulations are known or apparent to those skilled in the art, and are described in detail in *Remington's Pharmaceutical Sciences,* 1990 (above); and *Pharmaceutical Dosage Forms and Drug Delivery Systems,* 6th ed., Williams & Wilkins (1995).

In order to enhance the percutaneous absorption of the active ingredients, one or more of a number of agents can be added in the topical formulations, including but not limited to dimethylsulfoxide, dimethylacetamide, dimethylformamide, surfactants, azone, alcohol, acetone, propylene glycol and polyethylene glycol. In addition, physical methods can also be used to enhance transdermal penetration such as, *e.g*., by iontophoresis or sonophoresis. Alternatively, or in addition, liposomes may be employed.

The pharmaceutical compositions can be applied directly to the skin. Alternatively, they can be delivered by various transdermal drug delivery systems, such as transdermal patches as known in the art.

The invention having been described, the following examples are offered by way of illustration and not limitation.

### 6. Example: Targeting Function Screen

In this example, the effect of P protein on cellular targeting of tyrosinase was investigated. This function was then exploited in a screen for compounds that inhibit the activity of P protein.

### 6.1 Materials and Methods

Melan-a cells (*a*/*a*, *P*/*P*), an immortalized melanocyte line derived from C57BLI6J mice wildtype at the p locus (Bennett *et al*., 1987, Int. J. Cancer 39:414-418), were maintained in culture in Dulbecco's modification of Eagle's medium (DME). Melan-p1 melanocytes from mice lacking all p gene transcripts due to the presence of overlapping deletions (*a*/*a*, p^{cp}/p^{25H}) (Sviderskaya *et al*., 1997, J. Invest. Dermatol. 108:30-34) were maintained in Ham's F10 medium. Both media were supplemented with 10% fetal calf serum, 5% sodium pyruvate, 5% glutamate, 5 units/ml penicillin, 5 µg/ml streptomycin, 1% non-essential amino acids and 200 nM 12-0-tetradecanoyl phorbol 13-acetate. In addition, 200 pM cholera toxin was added to the melan-p1 cells.

Cells were maintained in the appropriate media, which was then replaced with tyrosine deficient DME medium (DME-D) supplemented with either 0.03 mM tyrosine for low tyrosine conditions or 0.3 mM tyrosine for high tyrosine conditions (Bennett, D.C. *et al*., 1987, Int. J. Cancer 39:414-418), (Sviderskaya *et al*., , J. Invest. Dermatol. 108:30-34). Aliquots of culture medium were withdrawn, dialyzed against 0.1 M sodium phosphate buffer, pH 6.8, and analyzed for tyrosinase activity using a radiometric tyrosine hydroxylase assay (Orlow, S.J. *et al.*, 1990, J. Invest. Dermatol. 94:461-64).

For treatment with test compounds, cultured melan-a melanocytes were incubated for 48 hours in the presence of low tyrosine in the medium as above but in the presence of benztropine (10 micromolar final concentration), or imipramine (10 micromolar final concentration), or nitroquipazine, (30 micromolar final concentration), or left untreated. Incubation media were assayed for tyrosinase activity, as above.

### 6.2 Results

Increasing tyrosinase activity in the media removed from melan-p cell cultures grown in the presence of low tyrosine indicates that these cells secrete relatively large amounts of tyrosinase into their incubation media (FIG. 1). By contrast, melan-a cells, which represent wildtype melanocytes, secrete significantly less tyrosinase into the media (FIG. 1). While culture in the presence of excess tyrosine had little effect on melan-a cells, the amount of enzyme secreted by melan-p1 cells was reduced. As predicted above, tyrosine appears to partially correct the misrouting of tyrosinase in melan-p1 cells.

Treatment with benztropine did not alter the levels of tyrosinase activity secreted to the incubation medium of melan-a cells (FIG. 2). Treatment with either imipramine or nitroquipazine significantly increased the levels of tyrosinase activity found in the cells' incubation medium (FIG. 2).

### 6.3 Discussion

Melan-a cells are melanocytes derived from wildtype mice. They have fully functional P protein and tyrosinase, and produce melanin. Melan-p cells, however, are derived from p-null mice having a deletion of the entire *p* gene coding sequence. Thus, they produce no P protein. Consequently, melan-p cells have lower tyrosinase activity and make less melanin than melan-a cells.

This example, which can be performed with any type of melanogenic cell, demonstrates that melanocytes lacking P protein function secrete significantly more tyrosinase into their growth or incubation medium than do melanocytes with normal P protein function. This result is obtained either when the cells are genetically altered to reduce or eliminate P protein function, as in melan-p cells (FIG. 1), or when the cells are treated with a compound that inhibits P protein function, such as imipramine (FIG. 2b).

### 7. Example: Tyrosinase Activity Screen

In this example, the effect of P protein on the measurable enzymatic activity of tyrosinase from cells genetically engineered to express tyrosinase was investigated. Any melanogenic cell type that expresses both P protein and tyrosinase, or any cell type made to express both P protein and tyrosinase, can be substituted. This function was then exploited in a screen for compounds that inhibit the function of P protein.

### 7.1 Materials and Methods

Cultured melan-a melanocytes, as described above in Section 6, were incubated for 48 hours in the presence of benztropine (10 micromolar final concentration), or imipramine (10 micromolar final concentration), or nitroquipazine (30 micromolar final concentration), or left untreated. Cells were washed and extracted with 50mM Tris-HCl (pH 7.4), 2mM EDTA, 150 mM NaCl and 1% Triton X-100. Cell extracts were analyzed for tyrosinase activity using a radiometric tyrosine hydroxylase assay (Orlow, S.J. *et al*., 1990, above).

Expression vectors were constructed to express P protein and tyrosinase genes in cultured cells. Specifically, the coding sequence for tyrosinase was removed as a HindIII-EcoRl fragment from clone TYBS (Yokohama *et al*., 1990, Nucl. Acids. Res. 18:7293-7298) and cloned into the HindIII/EcoRI sites of pcDNA l/amp (Invitrogen, CA). Coding sequence for the P protein was removed as a BamHI-EcoRV fragment from MC2701 (Gardner *et al*., 1992, above) and cloned into the BamHI/EcoRV sites of pcDNA3 and pcDNA3.1/V5/His-TOPO (Invitrogen, CA). COS cells were transfected with the pcDNA1-based plasmids and FuGENE™ 6 (Roche Molecular Biochemicals, Indianapolis, IN) as transfection agents for 48 hours. Cells were transformed with: (i) the vector alone; (ii) the vector carrying a tyrosinase-encoding gene; (iii) the vector carrying a P protein-encoding gene; or (iv) vectors carrying a tyrosinase-encoding gene and a P protein-encoding gene. Transformed cells were washed and extracted as above. Tyrosinase activity was then measured as above. Tyrosinase assays were performed on 60 micrograms of cell protein.

COS cells transfected with a vector carrying a tyrosinase-encoding gene, or with vectors carrying a tyrosinase-encoding gene and a P protein-encoding gene as above, were treated with benztropine, or imipramine, or nitroquipazine, or left untreated, as above, and cell extracts were then prepared as above. The tyrosinase activity of cell extracts was determined as above.

### 7.2 Results

As shown in FIG. 2a, extracts from melan-a cells treated with benztropine or nitroquipazine had greater tyrosinase activities than untreated cells. Extracts from cells treated with imipramine had less tyrosinase activity than untreated cells.

As shown in FIG. 3, extracts from COS cells transfected with the vector alone (V+V) or with the vector carrying the P protein-encoding gene (V+P) did not exhibit measurable tyrosinase activity. Extracts from cells transfected with the vector carrying the tyrosinase-encoding gene (V+T) had measurable tyrosinase activity, while extracts from cells transfected with the vectors carrying the tyrosinase-encoding gene and the P protein-encoding gene (T+P) had tyrosinase activity approximately four fold greater than the tyrosinase activity found in extracts of cells transfected with the vector carrying the tyrosinase-encoding gene alone (V+T).

FIG. 4 shows the separate effects of three compounds on P protein function. Nitroquipazine (4) caused extracts from tyrosinase-expressing COS cells to exhibit lower tyrosinase activity, regardless of whether the cells were expressing the P protein. Benztropine (2) did not have an appreciable effect on tyrosinase activity in these extracts. Imipramine (3) dramatically reduced the tyrosinase activity of cells expressing both P protein and tyrosinase, but had very little effect on cells expressing only tyrosinase.

### 7.3 Discussion

This example illuminates the relationship between P protein function and tyrosinase activity in cell extracts. Melanocytes that express P protein can be made to mimic cells that lack P protein function through the use of compounds that inhibit P protein function. Melan-a cells are wildtype for the P protein-encoding gene. Yet extracts taken from these cells after they are treated with imipramine have lower tyrosinase activity than untreated melan-a cells (FIG. 2). In contrast, extracts from cells treated with benztropine or nitroquipazine have higher tyrosinase activity than untreated cells (FIG. 2).

COS cells are derived from monkey kidney cells. Normally, they do not express tyrosinase or P protein. This example demonstrates that by transfecting COS cells with a tyrosinase-encoding gene and a P protein encoding gene, one can produce what might be considered an "artificial melanocyte." These cells express active tyrosinase and P protein (FIG. 3), and even produce melanin. Cotransfection of COS cells with both a tyrosinase-encoding gene and a P protein-encoding gene produces cells with approximately four times more tyrosinase activity than COS cells transfected with a tyrosinase-encoding gene alone (FIG. 3). This result demonstrates that P protein is expressed and active in these cells because the intracellular activity of tyrosinase was increased by P protein expression.

Extracts from COS cells that have been transformed with both a tyrosinase-encoding gene and a P protein-encoding gene and then treated with imipramine contained only about one third of the tyrosinase activity of similar cells not treated with imipramine (FIG. 4). The tyrosinase activity of COS cells that were transfected with only a tyrosinase-encoding gene and then treated with imipramine was not significantly different than the tyrosinase activity of extracts of similar cells not treated with imipramine (FIG. 4). These results indicate that imipramine reduces tyrosinase activity by inhibiting P protein function. By contrast, benztropine did not reduce the tyrosinase activity of extracts of transfected COS cells, whether or not they expressed P protein (FIG. 4). In addition, nitroquipazine reduced the tyrosinase activity of extracts of transfected COS cells, whether or not they expressed P protein (FIG. 4). This result indicates that nitroquipazine is not an inhibitor of P protein function.

### 8. Example: Secretion of Tyrosinase in Melan-p Cells Results from Proteolysis

While we observed activity of tyrosinase in the medium, Potterf *et al*. (1998, Exp. Cell Res. 244:319-326) did not detect tyrosinase protein in the medium using αPEP7. Tyrosinase is a type I membrane protein anchored in the membrane, and it is thus likely that proteolysis, which leads to the clipping of the tail, is required for secretion. The truncated protein would not be detected by αPEP7, which is directed against the tail, although the catalytic domains would remain functional. We therefore, examined the effects of a series of protease inhibitors on the secretion of tyrosinase by melan-a and melan-p1 cells. E64, an epoxysuccinyl peptide and a potent inhibitor of cysteine proteinases was found to be the most effective in reducing the amount of tyrosinase secreted into the media of melan-p1 cells (FIG. 5a), thus demonstrating that secretion of tyrosinase can be inhibited by blocking the activity of cysteinyl proteases.

If proteolysis and secretion of tyrosinase were the precipitating factor in the misrouting of tyrosinase, then E64 should increase melanin accumulation in melan-p1 cells. The effects of E64 were further investigated, and a potential synergy with tyrosine, which also reduced secretion into the media, examined. A range of E64 concentrations was tested at low (0.03 mM) and high (0.3 mM) tyrosine.

At 0.03 mM tyrosine, 12.5 µM E64 lowered secretion of tyrosinase into the medium from 7.1% to 4.0% (FIG. 5a), whereas at higher concentrations (25 µM), E64 was only slightly more effective (3.8% activity in media). E64 also reduced tyrosinase secretion at higher tyrosine concentrations (0.3 mM), reducing the tyrosinase in the medium from 6.5% to 3.9%. The higher concentration of E64 was not more effective. Surprisingly, E64 reduced intracellular melanin production at high concentrations of tyrosine. Thus, despite its ability to diminish proteolysis and secretion of tyrosinase from melan-p1 cells, E64 was not able to cause tyrosinase to re-route to the melanosome and begin melanin synthesis and deposition.

### 9. Example: Comparison of Ultrastructure and Distribution of Tyrosinase in Melan-a and Melan-p1 Cells

### 9.1 Materials and Methods

Melanocytes were seeded into Lab-Tek chamber slides (Nunc, Inc., Naperville, II) and grown to 90% confluence. Cultured melanocytes were fixed in wells with half-strength Karnovsky's fixative (Karnovsky, 1965) in 0.2 M sodium cacodylate buffer at pH 7.2 for 30 minutes at room temperature. For dihydroxyphenylalanine (DOPA) histochemistry, fixed cells were incubated in 0.1 % 1-DOPA twice for 2.5 hours. The cells were washed 3 times in buffer and treated with 1.0% osmium tetroxide containing 1.5% potassium ferrocyanide (Karnovsky, 1971) for 30 minutes. The cells were washed, stained en bloc with 0.5% uranyl acetate for 30 minutes, dehydrated, and embedded in Eponate 12. Areas of the Epon case were cut out and mounted on Epon pegs and sectioned on an RMC MT 6000-XL ultramicrotome. Ultrathin sections were stained with aqueous solutions of uranyl acetate (2%) and lead citrate (0.3%) for 15 minutes each, and then viewed and photographed in a JEOL JEM-IOOCX transmission electron microscope.

### 9.2 Results

Previous studies have shown that deficiency of the P protein results in both ultrastructural aberrations (Moyer, 1966, Am Zool 6:43-66; Sidman and Pearlstein, 1965, Dev. Biol. 12:93-116; Orlow and Brilliant, 1999, Exp. Eye Res. 68:147-154) as well as abnormal subcellular localization of tyrosinase (Potterf et al., 1998, *supra*)*.* In order to investigate both features simultaneously, we determined the subcellular architecture and the distribution of tyrosinase in melan-a and melan-p1 cells by electron microscopy with and without DOPA histochemistry.

As reported previously (Rosemblat *et al*., 1998, Exp. Cell Res. 239:344-352), cultured melan-a cells, wildtype at the *p* locus, contained melanosomes that were predominantly of stage IV maturation (FIG. 6a). In contrast, *p*-null melan-p1 cells exhibited melanosomes that were predominantly stage I and II and occasionally stage III (FIG. 6b).

Upon DOPA incubation of melan-a cells, tyrosinase activity was demonstrated in the trans Golgi network (TGN) and in 50 nm vesicles which were confined to the vicinity of the Golgi apparatus (FIG. 7a). DOPA treated melan-p1 cells also demonstrated reaction product in the TGN and neighboring 50 nm vesicles (FIG. 7b). In addition, reaction product was present in some melan-p1 melanosomes. However, many melanosomes, both in the cell body as well as in the dendrites, remained devoid of reaction product (FIG. 7b). Unlike melan-a cells (FIG. 7a), melan-p1 cells exhibited reaction product in 50 nm vesicles well outside of the peri-nuclear Golgi area (FIG. 7b) and in close proximity to the plasma membrane (FIG. 7b) suggesting an abnormal accumulation of tyrosinase in a population of vesicles.

### 9.3 Discussion

The lack of P protein resulted in the proliferation of small tyrosinase-containing vesicles that were no longer limited to the area around the TGN. Tyrosinase was therefore either packaged into different vesicles in the two cell lines or, alternatively, the vesicles were the same, but their routing was disrupted in the absence of P. Tyrosinase in these aberrant vesicles could be detected by DOPA staining and was thus enzymatically active, The increase in mature melanosomes in melan-p1 cells cultured in high tyrosine was not accompanied by a major reduction in the number of 50 nm vesicles, suggesting partial, but not complete, correction, of the p phenotype by tyrosine.

### 10. Example: Targeting of Lysosomal Hydrolases in Melan-a and Melan-p Cells

This experiment demonstrates that melan-p cells do not properly target a certain class of lysosomal hydrolases to the lysosome.

### 10.1 Material and Methods

Melan-a and melan-p cells as described above in Section 6 were seeded to high density and grown in low tyrosine (14 µM) DME medium. Large granule and small granule fractions were prepared and centrifuged on pre-layered sucrose gradients as described in Rosemblatt *et al*., 1994, above and Seiji, 1963, Annals N.Y. Acad. Sci., 100:497-533. Fractions were collected from the top down.

Appropriate reaction substrates for the lysosomal enzyme assays prepared in 0.2 M sodium acetate, 1% TritonX-100 were as follows:

| | |
|---|---|
| β-hexosaminidase | - 4 mM 4-methylumbelliferyl-N-acetyl-β-D-glucosaminide |
| β-glucosidase | - 4.6 mM 4-methylumbelliferyl-N-acetyl-β-D-glucoside |
| β-glucuronidase | - 4.6 mM 4-methylumbelliferyl-N-acetyl-β-D-glucoronide |
| β-galactosidase | - 4.6 mM 4-methylumbelliferyl-N-acetyl-β-D-galactoside |
| Acid phosphatase | - 22.5 mM 4-methylumbelliferyl-phosphate |

The reaction mix was prepared in 96 well flat bottom plates. Each well was loaded with 25 µl of a gradient fraction, 2.5 µl 1M sodium acetate and 27.5 µl of the appropriate substrate mix. The plates were covered with parafilm and incubated at 37°C. β-hexosaminidase reactions were incubated for 50 minutes, β-glucosidase, β-glucuronidase and β-galactosidase reactions were incubated for 20 minutes, and acid phosphatase reactions were incubated for 10 minutes. Reaction was stopped by addition of 200 µl of stop buffer (132 mM glycine, 68 mM sodium chloride, 83 mM anhydrous sodium carbonate), and plates read immediately using an excitation wavelength of 370 nm and an emission wavelength of 460 nm.

### 10.2 Results

In both melan-a and melan-p cells, very little of the lysosomal hydrolases were detected in the small granule fraction (see FIGS. 8-12). This result was expected because the small granule fraction consisted of mostly small vesicles in which lysosomal hydrolases do not normally accumulate. The large granule fraction contains endoplasmic reticulum, Golgi organelles, lysosomes and melanosomes and, hence, should contain most of the lysosomal hydrolases. With respect to acid phosphatase, there was only slightly less overall activity for the enzyme in large granule fractions from melan-p cells as compared to those from melan-a cells (FIG. 8B). Additionally, there was a minor shift in localization of acid phosphatase to slightly less dense fractions in the melan-p cells as compared to melan-a cells. However, with respect to the other lysosomal hydrolases assayed, the differences between melan-a and melan-p cells was dramatic. In fact, the overall activity of β-hexosaminidase, β-glucosidase, β-glucuronidase and β-galactosidase was significantly-reduced in melan-p cells as opposed to melan-a cells (see FIGS. 9-12, right panels). This loss of activity could not be attributed to a shift of the enzymes within the cells because whole cell extracts demonstrated the similar significant decreases in activity of β-hexosaminidase, β-glucosidase, β-glucuronidase and β-galactosidase in melan-p cells as opposed to melan-a cells, but with essentially no difference in the total amounts of alkaline phosphatase between melan-p and melan-a cells (results not shown). While the same large granule fractions from melan-a cells that contained acid phosphatase also contained most of the β-hexosaminidase, β-glucosidase, β-glucuronidase and β-galactosidase activities, melan-p cells had a significant reduction in activity of these enzymes in the large granule fractions. Thus, β-hexosaminidase, β-glucosidase, β-glucuronidase and β-galactosidase enzymes do not accumulate correctly in lysosomes in melan-p cells.

### 10.3 Discussion

Unlike acid phosphatase, the enzymes β-hexosaminidase, β-glucosidase, β-glucuronidase and β-galactosidase are not transported to the cell surface prior to eventually reaching the lysosome. Instead, these enzymes are transported from the *trans*-Golgi network to the late endosome via the activity of the M6P/IGF-II receptor. The differences in targeting of these two classes of lysosomal hydrolases in melan-p cells versus melan-a cells indicates that disruption of P protein function affects M6P/IGF-II receptor-mediated targeting. Based on our results showing the secretion of tyrosinase from melan-p cells, and the intracellular depletion of β-hexosaminidase, β-glucosidase, β-glucuronidase and β-galactosidase in large granule fractions from the same cells, this class of lysosomal enzymes should be secreted from the melan-p cells. Accordingly, targeting of these enzymes, assayed by an increase in secretion or a reduction in accumulation in lysosomal membrane fractions, can also be used as part of an assay to screen for compounds that affect the function of P protein.

### EQUIVALENTS

The foregoing written specification is sufficient to enable one skilled in the art to practice the invention.

## Claims

1. An in-vitro method of screening for compounds that inhibit melanogenesis, the method comprising: treating cells expressing a tyrosinase-encoding gene with a test compound, and determining the cellular localization of tyrosinase in the presence of the test compound; wherein a change in the cellular localization of tyrosinase in the presence of the test compound as compared to in the absence of the test compound indicates that the test compound is a candidate for a compound that inhibits melanogenesis.

2. The method of claim 1, wherein the cells further express a P protein-encoding gene, and wherein the change in the cellular localization of tyrosinase in the presence of the test compound as compared to in the absence of the test compound is dependent upon the expression of the P protein-encoding gene.

3. The method of claim 1 or 2, wherein the cellular localization of tyrosinase is determined by assaying the amount of tyrosinase secreted by the cells in the presence of the compound, wherein an increase in the amount of tyrosinase secreted by the cells in the presence of the test compound as compared to in the absence of the test compound indicates that the test compound is a candidate for a compound that inhibits melanogenesis.

4. The method of claim 1 or 2, wherein the cellular localization of tyrosinase is detected by assaying for tyrosinase activity.

5. The method of claim 1 or 2, wherein the cellular localization of tyrosinase is detected by assaying for the presence of tyrosinase protein using immunological techniques.

6. The method of claim 1 or 2 further comprising the step of assaying the amount of tyrosinase associated in a high molecular weight complex in the presence of the test compound, wherein a decrease in the amount of tyrosinase associated in a high molecular weight complex in the presence of the test compound as compared to in the absence of the test compound indicates that the test compound is a candidate for a compound that inhibits melanogenesis.

7. The method of claim 1 or 2 further comprising the step of assaying the amount of TRP-1 or TRP-2 protein associated in a high molecular weight complex in the presence of the compound, wherein a decrease in the amount of TRP-1 or TRP-2 protein associated in a high molecular weight complex in the presence of the test compound as compared to in the absence of the test compound indicates that the test compound is a candidate for a compound that inhibits melanogenesis.

8. The method of claim 1 or 2 further comprising the step of assaying the number or size of melanosomes in the cells in the presence of the compound, wherein a decrease in the number or size of melanosomes in the cells in the presence of the test compound as compared to in the absence of the test compound indicates that the test compound inhibits melanogenesis.

9. The method of claim 1 or 2 further comprising the step of assaying the mass or length of tyrosinase in the cells in the presence of the compound, wherein a decrease in the mass or length of tyrosinase in the cells in the presence of the test compound as compared to in the absence of the test compound indicates that the test compound is a candidate for a compound that inhibits melanogenesis.

10. The method of claim 1 or 2 further comprising the step of assaying for the levels and/or targeting of lysosomal hydrolases in the cells in the presence of the compound, wherein a decrease in accumulation of lysosomal hydrolases that are transported via the M6P/IGF-II receptor in the lysosome in the cells in the presence of the test compound as compared to in the absence of the test compound indicates that the test compound is a candidate for a compound that inhibits melanogenesis.

11. The method of claim 1 or 2, wherein the cells are grown in the presence of low tyrosine.

12. The method of claim 11 wherein the concentration of tyrosine is 0.01-0.03 mM.

13. The method of claim 1 wherein the cells are melanocytes.

14. The method of claim 1 wherein the cells are melanoma cells.

15. The method of claim 1 wherein the cells are derived from a mammal.

16. The method of claim 15 wherein the mammal is a human.

17. The method of claim 15 wherein the mammal is selected from the group consisting of mouse, hamster, and guinea pig.

18. An in-vitro method of screening for compounds that increase melanogenesis comprising: treating cells expressing a tyrosinase-encoding gene with a test compound, and determining the amount of tyrosinase secreted by the cells in the presence of the test compound; wherein a decrease in the amount of tyrosinase secreted by the cells in the presence of the test compound as compared to in the absence of the test compound indicates that the test compound is a candidate for a compound that increases melanogenesis.

19. The method of claim 18 wherein the cells further express a P protein-encoding gene, and wherein the decrease in the amount of tyrosinase secreted by the cells in the presence of the test compound as compared to in the absence of the test compound is dependent upon the expression of the P protein-encoding gene.

20. The method of claim 18 or 19 further comprising determining a ratio of tyrosinase inside the cells to tyrosinase secreted by the cells, wherein an increase in the ratio in the presence of the test compound as compared to in the absence of the test compound indicates that the test compound induces melanogenesis.

21. The method of claim 18 or 19, wherein the amount of tyrosinase is detected by assaying for tyrosinase activity.

22. The method of claim 18 or 19, wherein the amount of tyrosinase is detected by assaying for the presence of tyrosinase protein using immunological techniques.

23. The method of claim 18 wherein the cells are melanocytes.

24. The method of claim 18 wherein the cells are melanoma cells.

25. The method of claim 23 or 24, wherein the cells are visually examined for an increase in melanin production.

26. The method of claim 23 or 24 wherein the cells do not express P protein, and wherein a decrease in the amount of tyrosinase secreted by the cells in the presence of the test compound as compared to in the absence of the test compound indicates that the test compound is a candidate for a compound that mimics P protein function.

27. The method of claim 23 wherein the cells are mouse melan-p melanocytes.

28. The method of claim 18 wherein the cells are derived from a mammal.

29. The method of claim 28 wherein the mammal is a human.

30. The method of claim 28 wherein the mammal is selected from the group consisting of mouse, hamster, and guinea pig.

31. The method of claim 26, wherein the cells are visually examined for an increase in melanin production.

## Patentansprüche

1. In vitro-Verfahren zum Screening von Verbindungen, die eine Melanogenese inhibieren, wobei das Verfahren umfasst: Behandlung von Zellen, die ein Tyrosinase-kodierendes Gen exprimieren mit einer Testverbindung und Bestimmung der zellulären Lokalisation von Tyrosinase in der Gegenwart der Testverbindung; wobei eine Veränderung bei der zellulären Lokalisation von Tyrosinase in der Gegenwart der Testverbindung im Vergleich zu der Abwesenheit der Testverbindung darauf hinweist, dass die Testverbindung ein Kandidat ist für eine Verbindung, die eine Melanogenese inhibiert.

2. Verfahren nach Anspruch 1, wobei die Zellen weiter ein P-Proteinkodierendes Gen exprimieren und wobei die Veränderung bei der zellulären Lokalisation von Tyrosinase in der Gegenwart der Testverbindung im Vergleich zu der Abwesenheit der Testverbindung von der Expression des P-Protein-kodierenden Gens abhängt.

3. Verfahren nach Anspruch 1 oder 2, wobei die zelluläre Lokalisation von Tyrosinase bestimmt wird, indem die Menge von Tyrosinase, die von den Zellen in der Gegenwart der Verbindung sekretiert wird, untersucht wird, wobei ein Anstieg bei der Menge von Tyrosinase, die von den Zellen in der Gegenwart der Testverbindung im Vergleich zu der Abwesenheit der Testverbindung sekretiert wird, darauf hinweist, dass die Testverbindung ein Kandidat ist für eine Verbindung, die eine Melanogenese inhibiert.

4. Verfahren nach Anspruch 1 oder 2, wobei die zelluläre Lokalisation von Tyrosinase detektiert wird, indem die Tyrosinaseaktivität untersucht wird.

5. Verfahren nach Anspruch 1 oder 2, wobei die zelluläre Lokalisation von Tyrosinase detektiert wird, indem das Vorliegen von Tyrosinaseprotein unter Verwendung von immunologischen Techniken untersucht wird.

6. Verfahren nach Anspruch 1 oder 2, weiter umfassend den Schritt des Untersuchens der Menge von Tyrosinase, die mit einem Komplex mit hohem Molekulargewicht assoziiert ist in der Gegenwart der Testverbindung, wobei eine Abnahme in der Menge von Tyrosinase, die mit einem Komplex mit hohem Molekulargewicht assoziiert ist, in der Gegenwart der Testverbindung im Vergleich zu der Abwesenheit der Testverbindung darauf hinweist, dass die Testverbindung ein Kandidat ist für eine Verbindung, die eine Melanogenese inhibiert.

7. Verfahren nach Anspruch 1 oder 2, weiter umfassend den Schritt des Untersuchens der Menge von TRP-1- oder TRP-2-Protein, das mit einem Komplex mit hohem Molekulargewicht assoziiert ist in der Gegenwart der Verbindung, wobei eine Abnahme in der Menge von TRP-1- und TRP-2-Protein, das mit einem Komplex mit hohem Molekulargewicht assoziiert ist, in der Gegenwart der Testverbindung im Vergleich zu der Abwesenheit der Testverbindung darauf hinweist, dass die Testverbindung ein Kandidat ist für eine Verbindung, die Melanogenese inhibiert.

8. Verfahren nach Anspruch 1 oder 2, weiter umfassend den Schritt des Untersuchens der Anzahl oder der Größe von Melanosomen in den Zellen in der Gegenwart der Verbindung, wobei eine Abnahme in der Anzahl oder der Größe der Melanosome in den Zellen in der Gegenwart der Testverbindung im Vergleich zu der Abwesenheit der Testverbindung darauf hinweist, dass die Testverbindung eine Melanogenese inhibiert.

9. Verfahren nach Anspruch 1 oder 2, weiter umfassend den Schritt des Untersuchens der Masse oder der Länge der Tyrosinase in den Zellen in der Gegenwart der Verbindung, wobei eine Abnahme in der Masse oder der Länge der Tyrosinase in den Zellen in der Gegenwart der Testverbindung im Vergleich zu der Abwesenheit der Testverbindung darauf hinweist, dass die Testverbindung ein Kandidat ist für eine Verbindung, die eine Melanogenese inhibiert.

10. Verfahren nach Anspruch 1 oder 2, weiter umfassend den Schritt des Untersuchens der Spiegel und/oder der Ziele von lysosomalen Hydrolasen in den Zellen in der Gegenwart der Verbindung, wobei eine Abnahme bei der Anhäufung von lysosomalen Hydrolasen, die über den M6P/IGF-II-Rezeptor in das Lysosom in den Zellen transportiert werden, in der Gegenwart der Testverbindung im Vergleich zu der Abwesenheit der Testverbindung darauf hinweist, dass die Testverbindung ein Kandidat ist für eine Verbindung, die eine Melanogenese inhibiert.

11. Verfahren nach Anspruch 1 oder 2, wobei die Zellen in der Gegenwart von niedrigem Tyrosin gewachsen sind.

12. Verfahren nach Anspruch 11, wobei die Konzentration von Tyrosin 0,01-0,03 mM beträgt.

13. Verfahren nach Anspruch 1, wobei die Zellen Melanozyten sind.

14. Verfahren nach Anspruch 1, wobei die Zellen Melanomzellen sind.

15. Verfahren nach Anspruch 1, wobei die Zellen von einem Säugetier stammen.

16. Verfahren nach Anspruch 15, wobei das Säugetier ein Mensch ist.

17. Verfahren nach Anspruch 15, wobei das Säugetier ausgewählt ist aus der Gruppe bestehend aus Maus, Hamster und Meerschweinchen.

18. In vitro-Verfahren zum Screening von Verbindungen, die eine Melanogenese steigern, umfassend: Behandlung von Zellen, die ein Tyrosinase-kodierendes Gen exprimieren mit einer Testverbindung und Bestimmung der Menge von Tyrosinase, die von den Zellen in der Gegenwart der Testverbindung sekretiert wird; wobei eine Abnahme in der Menge der Tyrosinase, die durch die Zellen in der Gegenwart der Testverbindung im Vergleich zu der Abwesenheit der Testverbindung sekretiert wird, darauf hinweist, dass die Testverbindung ein Kandidat ist für eine Verbindung, die eine Melanogenese steigert.

19. Verfahren nach Anspruch 18, wobei die Zellen weiter ein P-Proteinkodierendes Gen exprimieren und wobei die Abnahme in der Menge von Tyrosinase, die von den Zellen in der Gegenwart der Testverbindung im Vergleich zu der Abwesenheit der Testverbindung sekretiert wird, von der Expression des P-Protein-kodierenden Gens abhängt.

20. Verfahren nach Anspruch 18 oder 19, weiter umfassend das Bestimmen eines Verhältnisses von Tyrosinase im Inneren der Zellen mit Tyrosinase, die von den Zellen sekretiert wird, wobei eine Zunahme in dem Verhältnis in der Gegenwart der Testverbindung im Vergleich zu der Abwesenheit der Testverbindung darauf hinweist, dass die Testverbindung eine Melanogenese induziert.

21. Verfahren nach Anspruch 18 oder 19, wobei die Menge von Tyrosinase detektiert wird, indem auf Tyrosinaseaktivität untersucht wird.

22. Verfahren nach Anspruch 18 oder 19, wobei die Menge von Tyrosinase detektiert wird, indem das Vorliegen von Tyrosinaseprotein unter Verwendung von immunologischen Techniken untersucht wird.

23. Verfahren nach Anspruch 18, wobei die Zellen Melanozyten sind.

24. Verfahren nach Anspruch 18, wobei die Zellen Melanomzellen sind.

25. Verfahren nach Anspruch 23 oder 24, wobei die Zellen visuell auf eine Zunahme bei der Melaninproduktion untersucht werden.

26. Verfahren nach Anspruch 23 oder 24, wobei die Zellen kein P-Protein exprimieren und wobei eine Abnahme bei der Menge von Tyrosinase, die von den Zellen in der Gegenwart der Testverbindung im Vergleich zu der Abwesenheit der Testverbindung sekretiert wird, darauf hinweist, dass die Testverbindung ein Kandidat ist für eine Verbindung, die eine P-Proteinfunktion nachahmt.

27. Verfahren nach Anspruch 23, wobei die Zellen Melan-P-Melanozyten der Maus sind.

28. Verfahren nach Anspruch 18, wobei die Zellen von einem Säugetier stammen.

29. Verfahren nach Anspruch 28, wobei das Säugetier ein Mensch ist.

30. Verfahren nach Anspruch 28, wobei das Säugetier ausgewählt ist aus der Gruppe bestehend aus Maus, Hamster und Meerschweinchen.

31. Verfahren nach Anspruch 26, wobei die Zellen auf eine Zunahme bei der Melaninproduktion visuell untersucht werden.

## Revendications

1. Procédé in vitro de criblage de composés qui inhibent la mélanogenèse, le procédé comprenant : le traitement de cellules exprimant un gène codant une tyrosinase avec un composé à tester, et la détermination de la localisation cellulaire de la tyrosinase en présence du composé à tester ; dans lequel un changement de la localisation cellulaire de la tyrosinase en présence du composé à tester par rapport à sa localisation en l'absence du composé à tester indique que le composé à tester est un composé candidat pour l'inhibition de la mélanogenèse.

2. Procédé de la revendication 1, dans lequel les cellules expriment en outre un gène codant une protéine P, et dans lequel le changement de la localisation cellulaire de la tyrosinase en présence du composé à tester par rapport à sa localisation en l'absence du composé à tester dépend de l'expression du gène codant la protéine P.

3. Procédé de la revendication 1 ou 2, dans lequel la localisation cellulaire de la tyrosinase est déterminée par test de la quantité de tyrosinase sécrétée par les cellules en présence du composé, dans lequel une augmentation de la quantité de tyrosinase sécrétée par les cellules en présence du composé à tester par rapport à sa sécrétion en l'absence du composé à tester indique que le composé à tester est un composé candidat pour l'inhibition de la mélanogenèse.

4. Procédé de la revendication 1 ou 2, dans lequel la localisation cellulaire de la tyrosinase est détectée par test de l'activité tyrosinase.

5. Procédé de la revendication 1 ou 2, dans lequel la localisation cellulaire de la tyrosinase est détectée par test de la présence de protéine tyrosinase en utilisant des techniques immunologiques.

6. Procédé de la revendication 1 ou 2, comprenant en outre l'étape de test de la quantité de tyrosinase associée dans un complexe de haut poids moléculaire en présence du composé à tester, dans lequel une diminution de la quantité de tyrosinase associée dans un complexe de haut poids moléculaire en présence du composé à tester par rapport à la quantité en l'absence du composé à tester indique que le composé à tester est un composé candidat pour l'inhibition de la mélanogenèse.

7. Procédé de la revendication 1 ou 2, comprenant en outre l'étape de test de la quantité protéine TRP-1 ou TRP-2 associée dans un complexe de haut poids moléculaire en présence du composé, dans lequel une diminution de la quantité de protéine TRP-1 ou de TRP-2 associée dans un complexe de haut poids moléculaire en présence du composé à tester par rapport à la quantité en l'absence du composé à tester indique que le composé à tester est un composé candidat pour l'inhibition de la mélanogenèse.

8. Procédé de la revendication 1 ou 2, comprenant en outre l'étape de test du nombre ou de la taille des mélanosomes dans les cellules en présence du composé à tester, dans lequel une diminution du nombre ou de la taille des mélanosomes dans les cellules en présence du composé à tester par rapport au nombre ou à la taille en l'absence du composé à tester indique que le composé à tester inhibe la mélanogenèse.

9. Procédé de la revendication 1 ou 2, comprenant en outre l'étape de test de la masse ou de la longueur de la tyrosinase dans les cellules en présence du composé, dans lequel une diminution de la masse ou de la longueur de la tyrosinase dans les cellules en présence du composé à tester par rapport à la masse ou à la longueur en l'absence du composé à tester indique que le composé à tester est un composé candidat pour l'inhibition de la mélanogenèse.

10. Procédé de la revendication 1 ou 2, comprenant en outre l'étape de test des teneurs et/ou du ciblage des hydrolases lysosomiales dans les cellules en présence du composé, dans lequel une diminution de l'accumulation des hydrolases lysosomiales qui sont transportées via le récepteur de M6P/IGF-II dans les cellules en présence du composé à tester par rapport à l'accumulation en l'absence du composé à tester indique que le composé à tester est un composé candidat pour l'inhibition de la mélanogenèse.

11. Procédé de la revendication 1 ou 2, dans lequel les cellules sont cultivées en présence d'une faible teneur en tyrosine.

12. Procédé de la revendication 11, dans lequel la concentration de tyrosine est de 0,01-0,03 mM.

13. Procédé de la revendication 1, dans lequel les cellules sont des mélanocytes.

14. Procédé de la revendication 1, dans lequel les cellules sont des cellules de mélanome.

15. Procédé de la revendication 1, dans lequel les cellules sont dérivées d'un mammifère.

16. Procédé de la revendication 1, dans lequel le mammifère est un humain.

17. Procédé de la revendication 15, dans lequel le mammifère est choisi dans l'ensemble consistant en souris, hamster et cochon d'Inde.

18. Procédé in vitro de criblage des composés qui augmentent la mélanogenèse, comprenant : le traitement de cellules exprimant un gène codant une tyrosinase avec un composé à tester, et la détermination de la quantité de tyrosinase sécrétée par les cellules en présence du composé à tester ; dans lequel une diminution de la quantité de tyrosinase sécrétée par les cellules en présence du composé à tester par rapport à la quantité en l'absence du composé à tester indique que le composé à tester est un composé candidat pour l'augmentation de la mélanogenèse.

19. Procédé de la revendication 18, dans lequel les cellules expriment en outre un gène codant une protéine P, et dans lequel la diminution de la quantité de tyrosinase sécrétée par les cellules en présence du composé à tester par rapport à la quantité en l'absence du composé à tester dépend de l'expression du gène codant la protéine P.

20. Procédé de la revendication 18 ou 19, comprenant en outre la détermination d'un rapport de tyrosinase à l'intérieur des cellules à la tyrosinase sécrétée par les cellules, dans lequel une augmentation du rapport en présence du composé à tester par rapport au rapport en l'absence du composé à tester indique que le composé à tester induit une mélanogenèse.

21. Procédé de la revendication 18 ou 19, dans lequel la quantité de tyrosinase est détectée par test de l'activité tyrosinase.

22. Procédé de la revendication 18 ou 19, dans lequel la quantité de tyrosinase est détectée par test de la présence de protéine tyrosinase en utilisant des techniques immunologiques.

23. Procédé de la revendication 18, dans lequel les cellules sont des mélanocytes.

24. Procédé de la revendication 18, dans lequel les cellules sont des cellules de mélanome.

25. Procédé de la revendication 23 ou 24, dans lequel les cellules sont examinées visuellement pour détecter une augmentation de la production de mélanine.

26. Procédé de la revendication 23 ou 24, dans lequel les cellules n'expriment pas la protéine P, et dans lequel une diminution de la quantité de tyrosinase sécrétée par les cellules en présence du composé à tester par rapport à la quantité en l'absence du composé à tester indique que le composé à tester est un composé candidat pour mimer la fonction de la protéine P.

27. Procédé de la revendication 23, dans lequel les cellules sont des mélanocytes melan-p de souris.

28. Procédé de la revendication 18, dans lequel les cellules sont dérivées d'un mammifère.

29. Procédé de la revendication 28, dans lequel le mammifère est un humain.

30. Procédé de la revendication 28, dans lequel le mammifère est choisi dans l'ensemble consistant en souris, hamster et cochon d'Inde.

31. Procédé de la revendication 26, dans lequel les cellules sont examinées visuellement pour détecter une augmentation de la production de mélanine.
